# EUROPEAN PATENT APPLICATION

(11) **EP 2 365 066 A1**
(43) Date of publication of application: **14.09.2011**
(21) Application number: 10012260.5
(22) Date of filing: 03.10.2006
(51) Int. Cl.: C12N 9/22, C12N 15/55, C12N 15/64, C12N 15/10, C12N 15/79, C12N 15/82

(54) **Laglidadg homing endonuclease variants having mutations in two functional subdominants und use thereof**

(30) Priority: 25.10.2005 WO PCT/IB2005/003568
(62) Divisional of application: 06842331.8
(71) Applicant: Cellectis, 93235 Romainville Cedex (FR)
(72) Inventor: Paques, Frédéric, 92340 Bourg-La-Reine (FR)
(74) Representative: Noel, Chantal Odile

(57) **Abstract**

A LAGLIDADG homing endonuclease variant, having mutations in two separate subdomains, each binding a distinct part of a modified DNA target half-site, said LAGLIDADG homing endonuclease variant being able to cleave a chimeric DNA target sequence comprising the nucleotides bound by each subdomain.

Use of said herodimeric meganuclease and derived products for genetic engineering, genome therapy and antiviral therapy.

## Description

The invention relates to a method for engineering a LAGLIDADG homing endonuclease variant, having mutations in two functional subdomains, each binding a distinct part of a modified DNA target half-site, said LAGLIDADG homing endonuclease variant being able to cleave a chimeric DNA target sequence comprising the nucleotides bound by each subdomain.

The invention relates also to a LAGLIDADG homing endonuclease variant obtainable by said method, to a vector encoding said variant, to a cell, an animal or a plant modified by said vector and to the use of said I-*Cre*I endonuclease variant and derived products for genetic engineering, genome therapy and antiviral therapy.

Meganucleases are by definition sequence-specific endonucleases with large (>14 bp) cleavage sites that can deliver DNA double-strand breaks (DSBs) at specific loci in living cells (Thierry and Dujon, Nucleic Acids Res., 1992, 20, 5625-5631). Meganucleases have been used to stimulate homologous recombination in the vicinity of their target sequences in cultured cells and plants (Rouet et al., Mol. Cell. Biol., 1994, 14, 8096-106; Choulika et al., Mol. Cell. Biol., 1995, 15, 1968-73; Donoho et al., Mol. Cell. Biol, 1998, 18, 4070-8; Elliott et al., Mol. Cell. Biol., 1998, 18, 93-101; Sargent et al., Mol. Cell. Biol., 1997, 17, 267-77; Puchta et al., Proc. Natl. Acad. Sci. USA, 1996, 93, 5055-60; Chiurazzi et al., Plant Cell, 1996, 8, 2057-2066), making meganuclease-induced recombination an efficient and robust method for genome engineering. The use of meganuclease-induced recombination has long been limited by the repertoire of natural meganucleases, and the major limitation of the current technology is the requirement for the prior introduction of a meganuclease cleavage site in the locus of interest. Thus, the making of artificial meganucleases with tailored substrate specificities is under intense investigation. Such proteins could be used to cleave genuine chromosomal sequences and open new perspectives for genome engineering in wide range of applications. For example, meganucleases could be used to induce the correction of mutations linked with monogenic inherited diseases, and bypass the risk due to the randomly inserted transgenes used in current gene therapy approaches (Hacein-Bey-Abina et al., Science, 2003, 302, 415-419).

Recently, Zinc-Finger DNA binding domains of Cys2-His2 type Zinc-Finger Proteins (ZFP) could be fused with the catalytic domain of the *Fok*I endonuclease, to induce recombination in various cell types, including human lymphoid cells (Smith et al., Nucleic Acids Res, 1999, 27, 674-81; Pabo et al., Annu. Rev. Biochem, 2001, 70, 3,13-40; Porteus and Baltimore, Science, 2003, 300, 763; Umov et al., Nature, 2005, 435, 646-651; Bibikova et al., Science, 2003, 300, 764). The binding specificity of ZFPs is relatively easy to manipulate, and a repertoire of novel artificial ZFPs, able to bind many (g/a)nn(g/a)nn(g/a)nn sequences is now available (Pabo et al., precited; Segal and Barbas, Curr. Opin. Biotechnol., 2001, 12, 632-7; Isalan et al., Nat. Biotechnol., 2001, 19, 656-60). However, preserving a very narrow specificity is one of the major issues for genome engineering applications, and presently it is unclear whether ZFPs would fulfill the very strict requirements for therapeutic applications. Furthermore, these fusion proteins have demonstrated high toxicity in cells (Porteus and Baltimore, precited; Bibikova et al., Genetics, 2002, 161, 1169-1175)), probably due to a low level of specificity.

In nature, meganucleases are essentially represented by homing endonucleases (HEs), a family of endonucleases encoded by mobile genetic elements, whose function is to initiate DNA double-strand break (DSB)-induced recombination events in a process referred to as homing (Chevalier and Stoddard, Nucleic Acids Res., 2001, 29, 3757-74; Kostriken et al., Cell; 1983, 35, 167-74; Jacquier and Dujon, Cell, 1985, 41, 383-94). Several hundreds of HES have been identified in bacteria, eukaryotes, and archea (Chevalier and Stoddard, precited); however the probability of finding a HE cleavage site in a chosen gene is very low.

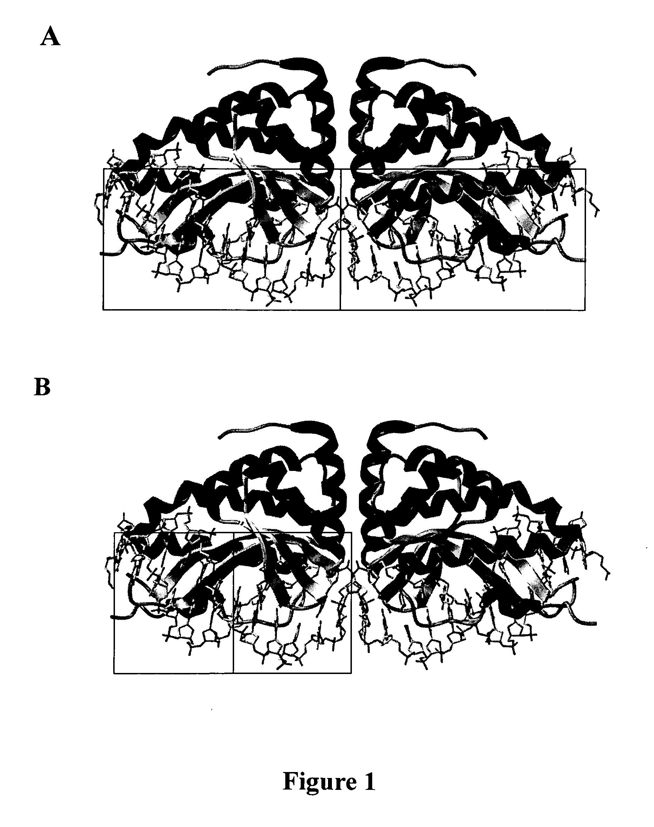

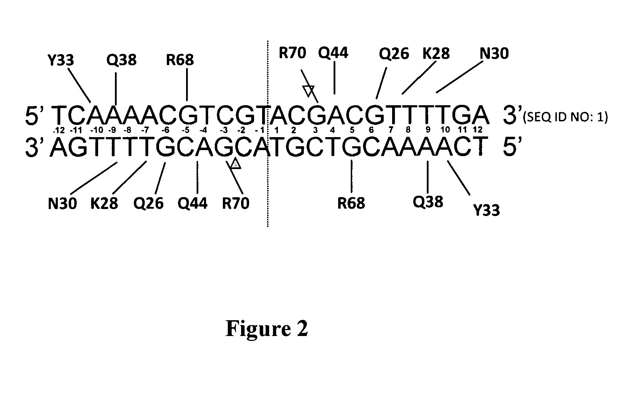

Given their biological function and their exceptional cleavage properties in terms of efficacy and specificity, HEs provide ideal scaffolds to derive novel endonucleases for genome engineering. Data have been accumulated over the last decade, characterizating the LAGLIDADG family, the largest of the four HE families (Chevalier and Stoddard, precited). LAGLIDADG refers to the only sequence actually conserved throughout the family and is found in one or (more often) two copies in the protein. Proteins with a single motif, such as I-*Cre*I; form homodimers and cleave palindromic or pseudo-palindromic DNA sequences, whereas the larger, double motif proteins, such as I-*Sce*I are monomers and cleave non-palindromic targets. Seven different LAGLIDADG proteins have been crystallized, and they exhibit a very striking conservation of the core structure, that contrasts with the lack of similarity at the primary sequence level (Jurica et al., Mol. Cell., 1998, 2, 469-76; Chevalier et al., Nat. Struct. Biol., 2001, 8, 312-6 ; Chevalier et al. J. Mol. Biol., 2003, 329, 253-69; Moure et al., J. Mol. Biol, 2003, 334, 685-95; Moure et al., Nat. Struct. Biol., 2002, 9, 764-70; Ichiyanagi et al., J. Mol. Biol., 2000, 300, 889-901; Duan et al., Cell, 1997, 89, 555-64; Bolduc et al., Genes Dev., 2003, 17, 2875-88; Silva et al., J. Mol. Biol., 1999, 286, 1123-36). In this core structure, two characteristic αββαββα folds, also called LAGLIDADG homing endonuclease core domains, contributed by two monomers, or by two domains in double LAGLIDAG proteins, are facing each other with a two-fold symmetry. DNA binding depends on the four β strands from each domain, folded into an antiparallel β-sheet, and forming a saddle on the DNA helix major groove (Figure 1A). Analysis of I-CreI structure bound to its natural target shows that in each monomer, eight residues (Y33, Q38, N30, K28, Q26, Q44, R68 and R70) establish direct interactions with seven bases at positions ± 3, 4, 5, 6, 7, 9 and 10 (Jurica et al., 1998, precited; figure 2). In addition, some residues establish water-mediated contact with several bases; for example S40, K28 and N30 with the base pair at position 8 and -8 (Chevalier et al., 2003, precited). The catalytic core is central, with a contribution of both symmetric monomers/domains. In addition to this core structure, other domains can be found: for example, PI-*Sce*I, an intein, has a protein splicing domain, and an additional DNA-binding domain (Moure et al., 2002, precited; Grindl et al., Nucleic Acids Res., 1998, 26, 1857-62).

Two approaches for deriving novel endonucleases from homing endonucleases, are under investigation:

### - protein variants

Altering the substrate specificity of DNA binding proteins by mutagenesis and screening/selection has often proven to be difficult (Lanio et al., Protein Eng., 2000, 13, 275-281; Voziyanov et al., J. Mol. Biol., 2003, 326, 65-76; Santoro et al., P.N.A.S., 2002, 99, 4185-4190; Buchholz and Stewart, Nat. Biotechnol., 2001, 19, 1047-1052), and more particularly, engineering HEs DNA binding domain has long been considered a daunting task (Ashworth et al., Nature 2006, 441, 656-659; Gimble et al., J. Mol. Biol., 2003, 334, 993-1008 ; Arnould et al., J. Mol. Biol., 2006, 355, 443-458; Doyon et al., J. Am. Chem. Soc., 2006, 128, 2477-2484; Steuer *et al.,* precited; Seligman et al., Nucleic Acids Res., 2002, 30, 3870-3879).

Analysis of the I-*Cre*I / DNA crystal structure indicates that 9 amino acids make direct contacts with the homing site (Chevalier *et al.,* 2003; Jurica et al., precited) which randomization would result in 20⁹ combinations, a number beyond any screening capacity today.

Therefore, several laboratories have relied on a semi-rational approach (Chica et al., Curr. Opin. Biotechnol., 2005, 16, 378-384) to limit the diversity of the mutant libraries to be handled: a small set of relevant residues is chosen according to structural data. Nevertheless, this was still not sufficient to create redesigned endonucleases cleaving chosen sequences:
- Seligman and co-workers used a rational approach to substitute specific individual residues of the I-*Cre*I αββαββα fold (Sussman et al., J. Mol. Biol., 2004, 342, 31-41; Seligman et al., Nucleic Acids Res., 2002, precited; Seligman et al., Genetics, 1997, 147, 1653-64); substantial cleavage was observed for few I-*Cre*I variants (Y33C, Y33H, Y33R, Y33L, Y33S, Y33T, S32K, S32R) and only for a target modified in position ±10.
- In a similar way, Gimble *et al.* (precited) modified the additional DNA binding domain of PI-*Sce*I; they obtained protein variants with altered binding specificity but no altered specificity and most of the variants maintained a lot of affinity for the wild-type target sequence.

### - hybrid or chimeric single-chain proteins

New meganucleases could be obtained by swapping LAGLIDADG homing endonuclease core domains of different monomers (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). These single-chain chimeric meganucleases wherein the two LAGLIDADG homing endonuclease core domains from different meganucleases are linked by a spacer, are able to cleave the hybrid target corresponding to the fusion of the two half parent DNA target sequences.

The construction of chimeric and single chain artificial HEs has suggested that a combinatorial approach could be used to obtain novel meganucleases cleaving novel (non-palindromic) target sequences: different monomers or core domains could be fused in a single protein, to achieve novel specificities. These results mean that the two DNA binding domains of an I-*Cre*I dimer behave independently; each DNA binding domain binds a different half of the DNA target site (Figure 1A). The generation of collections of novel meganucleases, and the ability to combine them by assembling two different monomers/core domains considerably enriches the number of DNA sequences that can be targeted, but does not yet saturate all potential sequences.

To reach a larger number of sequences, it would be extremely valuable to be able to identify smaller independent subdomains that could be combined (Figure 1B).

However, a combinatorial approach is much more difficult to apply within a single monomer or domain than between monomers since the structure of the binding interface is very compact and the two different ββ hairpins which are responsible for virtually all base-specific interactions do not constitute separate subdomains, but are part of a single fold. For example, in the internal part of the DNA binding regions of I-*Cre*I, the gtc triplet is bound by one residue from the first hairpin (Q44), and two residues from the second hairpin (R68 and R70; see figure 1B of Chevalier et al., 2003, precited). In addition the cumulative impact of a series of mutations could eventually disrupt proper folding.

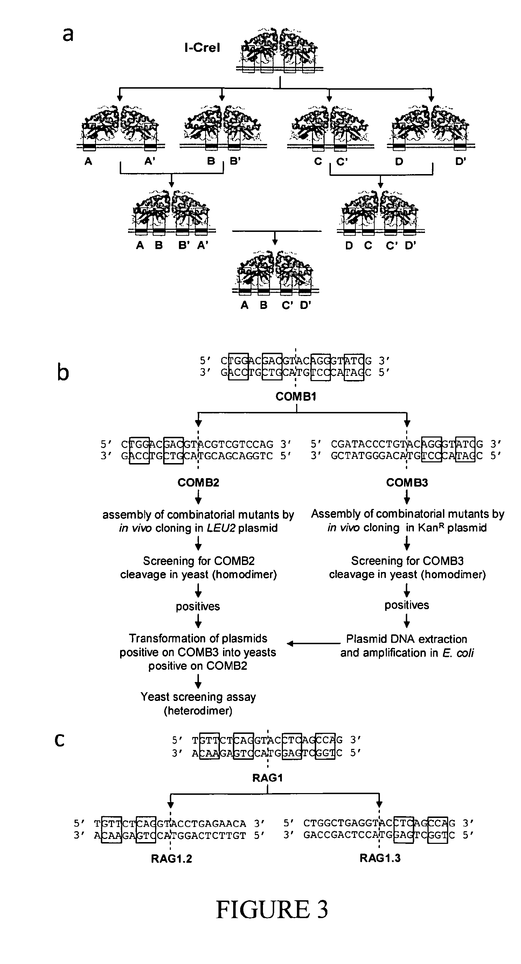

In spite of this lack of apparent modularity at the structural level, the Inventor has identified separable functional subdomains, able to bind distinct parts of a homing endonuclease half-site (figure 2). By assembling two subdomains from different monomers or core domains within the same monomer, the inventor has engineered functional homing endonuclease (homodimeric) variants, which are able to cleave palindromic chimeric targets (figure 3a). Furthermore, a larger combinatorial approach is allowed by assembling four different subdomains (figure 3a) to form new heterodimeric molecules which are able to cleave non-palindromic chimeric targets.

The different subdomains can be modified separately to engineer new cleavage specificities and the combination of different subdomains in one meganuclease (homodimer, heterodimer, single-chain chimeric molecule) increases considerably the number of DNA targets which can be cleaved by meganucleases. Thus, the identification of a small number of new cleavers for each subdomain allows for the design of a very large number of novel endonucleases with new specificities.

This approach was used to assemble four set of mutations into heterodimeric homing endonucleases with fully engineered specificity, to cleave a model target (COMB1) or a sequence from the human RAG1 gene. This is the first time a homing endonuclease is entirely redesigned to cleave a naturally occurring sequence.

Furthermore, in former studies, the targets of the engineered proteins differed from the initial wild-type substrate by 1 to 6 base pairs per site, whereas the 22 bp COMB1 and RAG1 sequences differ from the I-*Cre*I cleavage site (C1221) by 9 and 16 bp, respectively.

This new combinatioral approach which can be applied to any homing endonuclease (monomer with two domains or homodimer) considerably enriches the number of DNA sequences that can be targeted, resulting in the generation of dedicated meganucleases able to cleave sequences from many genes of interest. The generation of collections of I-*Cre*I derivatives and the ability to combine them intramolecularly as well as intermolecularly, increases the number of attainable 22-mer targets to at least 1.57 x 10⁷ ((64x62)²).

In addition, for genome engineering applications, the major advantage of HEs is their exquisite specificity, a feature that becomes essential when engaging into therapeutic applications.

Therefore, this approach provides a general method to create novel endonucleases cleaving chosen sequences. Potential applications include the cleavage of viral genomes specifically or the correction of genetic defects via double-strand break induced recombination, both of which lead to therapeutics.

The invention relates to a method for engineering a LAGLIDADG homing endonuclease variant derived from a parent LAGLIDADG homing endonuclease by mutation of two functional subdomains of the core domain, comprising at least the steps of :
(a) constructing a first variant having mutation(s) in a first functional subdomain of the core domain which interacts with a first part of one half of said parent LAGLIDADG homing endonuclease cleavage site, by:
   (a₁) replacing at least one amino acid of a first subdomain corresponding to that situated from positions 26 to 40 in I*-Cre*I*,* with a different amino acid,
   (a₂) selecting and/or screening the first variants from step (a₁) which are able to cleave a first DNA target sequence derived from said parent LAGLIDADG homing endonuclease half-site, by replacement of at least one nucleotide of said first part of the half-site, with a different nucleotide,
(b) constructing a second variant having mutation(s) in a second functional subdomain of the core domain which interacts with a second part of said parent LAGLIDADG homing endonuclease half-site, by:
   (b₁) replacing at least one amino acid of a second subdomain corresponding to that situated from positions 44 to 77 in I*-Cre*I, with a different amino acid,
   (b₂) selecting and/or screening the second variants from step (b₁) which are able to cleave a second DNA target sequence derived from said parent LAGLIDADG homing endonuclease half-site, by replacement of at least one nucleotide of said second part of the half-site, with a different nucleotide,
(c) constructing a third variant which has mutation(s) in the first and the second functional subdomains of said parent LAGLIDADG homing endonuclease, by:
   (c₁) combining the mutation(s) of two variants from step (a₁) and step (b₁) in a single variant, and
   (c₂) selecting and/or screening the variants from step (c₁) which are able to cleave a chimeric DNA target sequence comprising the first part of the first variant DNA target half-site and the second part of the second variant DNA target half-site.

### Definitions

- Amino acid residues in a polypeptide sequence are designated herein according to the one-letter code, in which, for example, Q means Gln or Glutamine residue, R means Arg or Arginine residue and D means Asp or Aspartic acid residue.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by "parent LAGLIDADG Homing Endonuclease" is intended a wild-type LAGLIDADG homing endonuclease or a functional variant thereof. Said parent LAGLIDADG Homing Endonuclease may be a monomer, a dimer (homodimer or heterodimer) comprising two LAGLIDADG Homing Endonuclease Core Domains which are associated in a functional endonuclease able to cleave a double-stranded DNA target of 22 to 24 bp.
- by "LAGLIDADG Homing Endonuclease variant" or "variant" is intended a protein obtained by replacing at least one amino acid of a LAGLIDADG Homing Endonuclease sequence, with a different amino acid.
- by "functional variant" is intended a LAGLIDADG Homing Endonuclease variant which is able to cleave a DNA target, preferably a new DNA target which is not cleaved by a wild-type LAGLIDADG Homing Endonuclease. For example, such variants have amino acid variation at positions contacting the DNA target sequence or interacting directly or indirectly with said DNA target.
- by "homing endonuclease variant with novel specificity" is intended a variant having a pattern of cleaved targets different from that of the parent homing endonuclease. The terms "novel specificity", "modified specificity", "novel cleavage specificity", "novel substrate specificity" which are equivalent and used indifferently, refer to the specificity of the variant towards the nucleotides of the DNA target sequence.
- by "I*-Cre*I" is intended the wild-type I-*Cre*I having the sequence SWISSPROT P05725 or pdb accession code 1g9y.
- by "domain" or "core domain" is intended the "LAGLIDADG Homing Endonuclease Core Domain" which is the characteristic α₁β₁β₂α₂β₃β₄α₃ fold of the homing endonucleases of the LAGLIDADG family, corresponding to a sequence of about one hundred amino acid residues. Said domain comprises four beta-strands (β₁, β₂, β₃, β₄) folded in an antiparallel beta-sheet which interacts with one half of the DNA target. This domain is able to associate with another LAGLIDADG Homing Endonuclease Core Domain which interacts with the other half of the DNA target to form a functional endonuclease able to cleave said DNA target. For example, in the case of the dimeric homing endonuclease I-*Cre*I (163 amino acids), the LAGLIDADG Homing Endonuclease Core Domain corresponds to the residues 6 to 94. In the case of monomeric homing endonucleases, two such domains are found in the sequence of the endonuclease; for example in I*-Dmo*I (194 amino acids), the first domain (residues 7 to 99) and the second domain (residues 104 to 194) are separated by a short linker (residues 100 to 103).
- by "subdomain" is intended the region of a LAGLIDADG Homing Endonuclease Core Domain which interacts with a distinct part of a homing endonuclease DNA target half-site. Two different subdomains behave independently and the mutation in one subdomain does not alter the binding and cleavage properties of the other subdomain. Therefore, two subdomains bind distinct part of a homing endonuclease DNA target half-site.
- by "beta-hairpin" is intended two consecutive beta-strands of the antiparallel beta-sheet of a LAGLIDADG homing endonuclease core domain (β₁β₂ or, β₃β₄ ) which are connected by a loop or a turn,
- by "DNA target", "DNA target sequence", "target sequence" , "target-site", "target" , "site"; "recognition site", "recognition sequence", "homing recognition site", "homing site", "cleavage site" is intended a 22 to 24 bp double-stranded palindromic, partially palindromic (pseudo-palindromic) or non-palindromic polynucleotide sequence that is recognized and cleaved by a LAGLIDADG homing endonuclease. These terms refer to a distinct DNA location, preferably a genomic location, at which a double stranded break (cleavage) is to be induced by the endonuclease. The DNA target is defined by the 5' to 3' sequence of one strand of the double-stranded polynucleotide. For example, the palindromic DNA target sequence cleaved by wild-type I*-Cre*I presented in figure 2 is defined by the sequence 5'- t₋₁₂c₋₁₁a₋₁₀a₋₉a₋₈a₋₇c₋₆g₋₅t₋₄c₋₃g₋₂t₋₁₋a₊₁c₊₂g₊₃a₊₄c₊₅g₊₆t₊₇t₊₈t₊₉t₊₁₀g₊₁₁a₊₁₂ (SEQ ID NO :1).
- by " DNA target half-site", "half cleavage site" or half-site" is intended the portion of the DNA target which is bound by each LAGLIDADG homing endonuclease core domain.
- by "chimeric DNA target", combined DNA target or "hybrid DNA target" is intended a DNA target, wherein at least one half of said target comprises the combination of nucleotides which are bound by at least two separate subdomains.
- by "vector" is intended a nucleic acid molecule capable of transporting another nucleic acid to which it has been linked.
- by "homologous" is intended a sequence with enough identity to another one to lead to a homologous recombination between sequences, more particularly having at least 95 % identity, preferably 97 % identity and more preferably 99 %.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or

BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and ruminants (e.g., cows, pigs, horses).
- "genetic disease" refers to any disease, partially or completely, directly or indirectly, due to an abnormality in one or several genes. Said abnormality can be a mutation, an insertion or a deletion. Said mutation can be a punctual mutation. Said abnormality can affect the coding sequence of the gene or its regulatory sequence. Said abnormality can affect the structure of the genomic sequence or the structure or stability of the encoded mRNA. Said genetic disease can be recessive or dominant. Such genetic disease could be, but are not limited to, cystic fibrosis, Huntington's chorea, familial hyperchoiesterolemia (LDL receptor defect), hepatoblastoma, Wilson's disease, congenital hepatic porphyrias, inherited disorders of hepatic metabolism, Lesch Nyhan syndrome, sickle cell anemia, thalassaemias, xeroderma pigmentosum, Fanconi's anemia, retinitis pigmentosa, ataxia telangiectasia, Bloom's syndrome, retinoblastoma, Duchenne's muscular dystrophy, and Tay-Sachs disease.

According to the method of the invention, each substitution is at the position of an amino acid residue which interacts with a DNA target half-site. The LAGLIDADG homing endonucleases DNA interacting residues are well-known in the art. The residues which are mutated may interact with the DNA backbone or with the nucleotide bases, directly or via a water molecule.

According to an advantageous embodiment of said method, the amino acid in step a₁) or b₁) is replaced with an amino acid which is selected from the group consisting of A, C, D, E, G, H, K, N, P, Q, R, S, T, L, V, W and Y.

According to another advantageous embodiment of said method, the amino acid which is replaced in step a₁) is situated from positions 28 to 40 in I*-Cre*I*.*

According to another advantageous embodiment of said method, the amino acid which is replaced in step b₁) is situated from positions 44 to 70 in I*-Cre*I*.*

According to the method of the invention, each part of the DNA target half-site comprises at least two consecutive nucleotides, preferably three consecutive nucleotides, and the first and the second part are separated by at least one nucleotide, preferably at least two nucleotides.

According to another advantageous embodiment of said method, the first and the second part of said half-site are situated in the external and the internal quarter of said half-site, respectively.

According to the method of the invention, the parent DNA target may be palindromic, non-palindromic or pseudo-palindromic.

According to the invention, the positions of the subdomains are defined by reference to I*-Cre*I structure (pdb accession code 1g9y). Knowing the positions of the subdomains in I*-Cre*I*,* one skilled in the art can easily deduce the corresponding positions in another LAGLIDADG homing endonuclease, using well-known protein structure analyses softwares such as Pymol. For example, for I*-Mso*I*,* the two functional subdomains are situated from positions 30 to 43 and 47 to 75, respectively.

According to the method of the invention, the amino acid mutation(s) in step a₁) or b₁) are introduced in either a wild-type LAGLIDADG homing endonuclease or a functional variant thereof.

The parent LAGLIDADG homing endonuclease may be selected from the group consisting of: I*-Sce*I, I*-Chu*I*,* I*-Cre*I, I*-Csm*I*,* PI-*Sce*I, PI-*Tli*I, PI*-Mtu*I, I*-Ceu*I, I*-Sce*II, I-*Sce* III, HO, PI-*Civ*I, PI*-Ctr*I, PI*-Aae*I*,* PI*-Bsu*I, PI*-Dha*I*,* PI*-Dra*I, PI-*Mav*I, PI*-Mch*I, PI-*Mfu*I, PI-*Mfl*I*,* PI*-Mga*I*,* PI-*Mgo*I, PI-Min*I*, PI-*Mka*I*,* PI-*Mle*I, PI*-Mma*I, PI*-Msh*I*,* PI*-Msm*I*,* PI*-Mth*I, PI*-Mtu*I*,* PI-*Mxe*I, PI*-Npu*I*,* PI*-Pfu*I, PI*-Rma*I, PI*-Spb*I, PI*-Ssp*I, PI*-Fac*I, PI-*Mja*I, PI*-Pho*I, PI*-Tag*I, PI*-Thy*I, PI*-Tk*I, PI*-Tsp*I*,* I*-Mso*I, and I*-Ani*I; preferably, I*-Cre*I, I*-Sce*I, I*-Chu*I, I*-Dmo*I, I*-Csm*I, PI*-Sce*I*,* PI-PfuI, PI-*Tli*I, PI*-Mtu*I, I*-Mso*I, I*-Ani*I and I-*Ceu*I; more preferably, I*-Cre*I, I*-Mso*I, I*-Sce*I, I-*Ani*I, I*-Dmo*I, PI*-Sce*I*,* and PI*-Pfu*I; still more preferably I*-Cre*I*.*

Functional variants comprise mutations that do not affect the protein structure. For example, the parent homing endonuclease may be an I-*Cre*I variant comprising one or more mutations selected from the group consisting of:
- the mutation of the isoleucine in position 24 in a valine (I24V),
- the mutation of the arginine in position 70, in a serine (R70S), and
- the mutation of the aspartic acid in position 75, in an uncharged amino acid, preferably an asparagine (D75N) or a valine (D75V),

Step a₁) or b₁) may comprise the introduction of additional mutations, particularly at other positions contacting the DNA target sequence or interacting directly or indirectly with said DNA target.

This step may be performed by generating a library of variants as described in the International PCT Application WO 2004/067736.

The combination of mutations in step c₁) may be performed by amplifying overlapping fragments comprising each of the two subdomains, according to well-known overlapping PCR techniques.

The selection and/or screening in step a₂), b₂) or c₂) may be performed by using a cleavage assay *in vitro* or *in vivo,* as described in the International PCT Application WO 2004/067736.

According to another advantageous embodiment of said method, step a₂), b₂), and/or c₂) are performed *in vivo,* under conditions where the double-strand break in the mutated DNA target sequence which is generated by said variant leads to the activation of a positive selection marker or a reporter gene, or the inactivation of a negative selection marker or a reporter gene, by recombination-mediated repair of said DNA double-strand break.

For example, the cleavage activity of the variant of the invention may be measured by a direct repeat recombination assay, in yeast or mammalian cells, using a reporter vector, as described in the PCT Application WO 2004/067736. The reporter vector comprises two truncated, non-functional copies of a reporter gene (direct repeats) and a chimeric DNA target sequence within the intervening sequence, cloned in a yeast or a mammalian expression vector. The chimeric DNA target sequence is made of the combination of the different parts of each initial variant half-site. Expression of the variant results in a functional endonuclease which is able to cleave the chimeric DNA target sequence. This cleavage induces homologous recombination between the direct repeats, resulting in a functional reporter gene, whose expression can be monitored by appropriate assay.

According to another advantageous embodiment of said method, it comprises a further step d₁) of expressing one variant obtained in step c₂), so as to allow the formation of homodimers. Said homodimers are able to cleave a palindromic or pseudo-palindromic chimeric target sequence comprising two different parts, each from one of the two initial variants half-sites (Figure 3a).

According to another advantageous embodiment of said method, it comprises a further step d'₁) of co-expressing one variant obtained in step c₂) and a wild-type LAGLIDADG homing endonuclease or a functional variant thereof, so as to allow the formation of heterodimers. Preferably, two different variants obtained in step c₂) are co-expressed. Said heterodimers are able to cleave a non-palindromic chimeric target sequence comprising four different parts (A, B, C', D'; Figure 3a), each from one of the four initial variants half-sites (two initial variants for each of the two different monomers; Figure 3a).

For example, host cells may be modified by one or two recombinant expression vector(s) encoding said variant(s). The cells are then cultured under conditions allowing the expression of the variant(s) and the homodimers/heterodimers which are formed are then recovered from the cell culture.

According to the method of the invention, single-chain chimeric endonucleases may be constructed by the fusion of one variant obtained in step c₂) with a homing endonuclease domain/monomer. Said domain/monomer may be from a wild-type homing endonuclease or a functional variant thereof.

Methods for constructing single-chain chimeric molecules derived from homing endonucleases are well-known in the art (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). Any of such methods, may be applied for constructing single-chain chimeric endonucleases derived from the variants as defined in the present invention.

The subject matter of the present invention is also a LAGLIDADG homing endonuclease variant obtainable by the method as defined above.

In a first preferred embodiment of said variant, it is an I-*Cre*I variant having at least two substitutions, one in each of the two subdomains situated from positions 26 to 40 and 44 to 77 of I-*Cre*I, respectively.

In a more preferred embodiment, said substitution(s) in the subdomain situated from positions 44 to 77 of I-*Cre*I are in positions 44, 68, 70, 75 and/or 77.

In another more preferred embodiment, said substitution(s) in the functional subdomain situated from positions 26 to 40 of I-*Cre*I are in positions 26, 28, 30, 32, 33, 38 and/or 40 of I*-Cre*I.

In another more preferred embodiment of said variant, it has at least one first substitution in positions 28 to 40 of I-*Cre*I and one second substitution in positions 44 to 70 of I*-Cre*I*.*

Preferably, said variant has amino acid residues in positions 44, 68 and 70, which are selected from the group consisting of: A44/A68/A70, A44/A68/G70, A44/A68/H70, A44/A68/K70, A44/A68/N70, A44/A68/Q70, A44/A68/R70, A44/A68/S70, A44/A68/T70, A44/D68/H70, A44/D68/K70, A44/D68/R70, A44/G68/H70, A44/G68/K70, A44/G68/N70, A44/G68/P70, A44/G68/R70, A44/H68/A70, A44/H68/G70, A44/H68/H70, A44/H68/K70, A44/H68/N70, A44/H68/Q70, A44/H68/R70, A44/H68/S70, A44/H68/T70, A44/K68/A70, A44/K68/G70, A44/K68/H70, A44/K68/K70, A44/K68/N70, A44/K68/Q70, A44/K68/R70, A44/K68/S70, A44/K68/T70, A44/N68/A70, A44/N68/E70, A44/N68/G70, A44/N68/H70, A44/N68/K70, A44/N68/N70, A44/N68/Q70, A44/N68/R70, A44/N68/S70, A44/N68/T70, A44/Q68/A70, A44/Q68/D70, A44/Q68/G70, A44/Q68/H70, A44/Q68/N70, A44/Q68/R70, A44/Q68/S70, A44/R68/A70, A44/R68/D70, A44/R68/E70, A44/R68/G70, A44/R68/H70, A44/R68/K70, A44/R68/L70, A44/R68/N70, A44/R68/R70, A44/R68/S70, A44/R68/T70, A44/S68/A70, A44/S68/G70, A44/S68/K70, A44/S68/N70, A44/S68/Q70, A44/S68/R70, A44/S68/S70, A44/S68/T70, A44/T68/A70, A44/T68/G70, A44/T68/H70, A44/T68/K70, A44/T68/N70, A44/T68/Q70, A44/T68/R70, A44/T68/S70, A44/T68/T70, D44/D68/H70, D44/N68/S70, D44/R68/A70, D44/R68/K70, D44/R68/N70, D44/R68/Q70, D44/R68/R70, D44/R68/S70, D44/R68/T70, E44/H68/H70, E44/R68/A70, E44/R68/H70, E44/R68/N70, E44/R68/S70, E44/R68/T70, E44/S68/T70, G44/H68/K70, G44/Q68/H70, G44/R68/Q70, G44/R68/R70, G44/T68/D70, G44/T68/P70, G44/T68/R70, H44/A68/S70, H44/A68/T70, H44/R68/A70, H44/R68/D70, H44/R68/E70, H44/R68/G70, H44/R68/N70, H44/R68/R70, H44/R68/S70, H44/R68/T70, H44/S68/G70, H44/S68/S70, H44/S68/T70, H44/T68/S70, H44/T68/T70, K44/A68/A70, K44/A68/D70, K44/A68/E70, K44/A68/G70, K44/A68/H70, K44/A68/N70, K44/A68/Q70, K44/A68/S70, K44/A68/T70, K44/D68/A70, K44/D68/T70, K44/E68/G70, K44/E68/N70, K44/E68/S70, K44/G68/A70, K44/G68/G70, K44/G68/N70, K44/G68/S70, K44/G68/T70, K44/H68/D70, K44/H68/E70, K44/H68/G70, K44/H68/N70, K44/H68/S70, K44/H68/T70, K44/K68/A70, K44/K68/D70, K44/K68/H70, K44/K68/T70, K44/N68/A70, K44/N68/D70, K44/N68/E70, K44/N68/G70, K44/N68/H70, K44/N68/N70, K44/N68/Q70, K44/N68/S70, K44/N68/T70, K44/P68/H70, K44/Q68/A70, K44/Q68/D70, K44/Q68/E70, K44/Q68/S70, K44/Q68/T70, K44/R68/A70, K44/R68/D70, K44/R68/E70, . K44/R681G70, K44/R68/H70, K44/R68/N70, K44/R68/Q70, K44/R68/570, K44/R68/T70, K44/S68/A70, K44/S68/D70, K44/S68/H70, K44/S68/N70, K44/S68/S70, K44/S68/T70, K44/T68/A70, K44/T68/D70, K44/T68/E70, K44/T68/G70, K44/T68/H70, K44/T68/N70, K44/T68/Q70, K44/T68/S70, K44/T68/T70, N44/A68/H70, N44/A68/R70, N44/H68/N70, N44/H68/R70, N44/K68/G70, N44/K68/H70, N44/K68/R70, N44/K68/S70, N44/N68/R70, N44/P68/D70, N44/Q68/H70, N44/Q68/R70, N44/R68/A70, N44/R68/D70, N44/R68/E70, N44/R68/G70, N44/R68/H70, N44/R68/K70, N44/R68/N70, N44/R68/R70, N44/R68/S70, N44/R68/T70, N44/S68/G70, N44/S68/H70, N44/S68/K70, N44/S68/R70, N44/T68/H70, N44/T68/K70, N44/T68/Q70, N44/T68/R70, N44/T68/S70, P44/N68/D70, P44/T68/T70, Q44/A68/A70, Q44/A68/H70, Q44/A68/R70, Q44/G68/K70, Q44/G68/R70, Q44/K68/G70, Q44/N681A70, Q44/N68/H70, Q44/N68/S70, Q44/P68/P70, Q44/Q68/G70, Q44/R68/A70, Q44/R68/D70, Q44/R68/E70, Q44/R68/G70, Q44/R68/H70, Q44/R68/N70, Q44/R68/Q70, Q44/R68/S70, Q44/S68/H70, Q44/S68/R70, Q44/S68/S70, Q44/T68/A70, Q44/T68/G70, Q44/T68/H70, Q44/T68/R70, R44/A68/G70, R44/A68/T70, R44/G68/T70, R44/H68/D70, R44/H68/T70, R44/N68/T70, R44/R68/A70, R44/R68/D70, R44/R68/E70, R44/R68/G70, R44/R68/N70, R44/R68/Q70, R44/R68/S70, R44/R68/T70, R44/S68/G70, R44/S68/N70, R44/S68/S70, R44/S68/T70, S44/D68/K70, S44/H68/R70, S44/R68/G70, S44/R68/N70, S44/R68/R70, S44/R68/S70, T44/A68/K70, T44/A68/R70, T44/H68/R70, T44/K68/R70, T44/N68/P70, T44/N68/R70, T44/Q68/K70, T44/Q68/R70, T44/R68/A70, T44/R68/D70, T44/R68/E70, T44/R68/G70, T44/R68/H70, T44/R68/K70, T44/R68/N70, T44/R68/Q70, T44/R68/R70, T44/R68/S70, T44/R68/T70, T44/S68/K70, T44/S68/R70, T44/T68/K70, and T44/T68/R70.

Preferably, said variant has amino acid in positions 28, 30, 33, 38 and 40 respectively, which are selected from the group consisting of: QNYKR, RNKRQ, QNRRR, QNYKK, QNTQK, QNRRK, KNTQR, SNRSR, NNYQR, KNTRQ, KNSRE, QNNQK, SNYRK, KNSRD, KNRER, KNSRS, RNRDR, ANSQR, QNYRK, QNKRT, RNAYQ, KNRQE, NNSRK, NNSRR, QNYQK, QNYQR, SNRQR, QNRQK, ENRRK, KNNQA, SNYQK, TNRQR, QNTQR, KNRTQ, KNRTR, QNEDH, RNYNA, QNYTR, RNTRA, HNYDS, QNYRA, QNYAR, SNQAA, QNYEK, TNNQR, QNYRS, KNRQR, QNRAR, QNNQR, RNRER, KNRAR, KNTAA, KNRKA, RNAKS, KNRNA, TNESD, RNNQD, RNRYQ, KNYQN, KNRSS, KNRYA, ANNRK, KNRAT, KNRNQ, TNTQR, KNRQY, QNSRK, RNYQS, QNRQR, KNRAQ, ANRQR, KNRQQ, KNRQA, KNTAS, KAHRS, KHHRS, KDNHS, KESRS, KHTPS, KGHYS, KARQS, KSRGS, KSHHS, KNHRS, KRRES, KDGHS, KRHGS, KANQS, KDHKS, KKHRS, KQNQS, KQTQS, KGRQS, KRPGS, KRGNS, KNAQS, KNHNS, KHHAS, KRGSS, KSRQS, KTDHS, KHHQS, KADHS, KSHRS, KNRAS, KSHQS, KDAHS, KNHES, KDRTS, KDRSS, KAHQS, KRGTS, KNHSS, KQHQS, KNHGS, KNNQS, KNDQS, KDRGS, KNHAS, KHMAS, KSSHS, KGVAS, KSVQS, KDVHS, RDVQS, KGVQS, KGVTS, KGVHS, KGVRS, KGVGS, RAVGS, RDVRS, RNVQS, and NTVDS.

In another more preferred embodiment, said variant cleaves a chimeric DNA target comprising a sequence having the formula : c₋₁₁n₋₁₀n₋₉n₋₈m₋₇y₋₆n₋₅n₋₄n₋₃k₋₂y₋₁r₊₁m₊₂n₊₃n₊₄n₊₅r₊₆k₊₇n₊₈n₊₉n₊₁₀g₊₁₁ (I) ,
wherein n is a, t, c, or g, m is a or c, y is c or t, k is g or t, r is a or g (SEQ ID NO: 2), providing that when n₋₁₀n₋₉n₋₈ is aaa and n₋₅n₋₄n₋₃ is gtc then n₊₈n₊₉n₊₁₀ is different from ttt and n₊₃n₊₄n₊₅ is different from gac and when n+₈n₊₉n₊₁₀ is ttt and n+₃n+₄n₊₅ is gac then n₋₁₀n₋₉n₋₈ is different from aaa and n₋₅n₋₄n₋₃ is different from gtc.

According to the invention, said chimeric DNA target may be palindromic, pseudopalindromic or non-palindromic. Preferably, the nucleotide sequence from positions -11 to -8 and +8 to +11 and/or the nucleotide sequence from positions -5 to -3 and/or +3 to +5 are palindromic.

More preferably, for cleaving a chimeric DNA target, wherein n₋₄ is t or n₊₄ is a, said variant has a glutamine (Q) in position 44.

More preferably, for cleaving a chimeric DNA target, wherein n₋₄ is a or n₊₄ is t, said variant has an alanine (A) or an asparagine in position 44; the I-*Cre*I variants comprising A44, R68, S70 or A44, R68, S70, N75 are examples of such variants.

More preferably, for cleaving a chimeric DNA target, wherein n₋₄ is c or n₊₄ is g, said variant has a lysine (K) in position 44; the I-*Cre*I variants comprising K44, R68, E70 or K44, R68, E70, N75 are examples of such variants.

More preferably, for cleaving a chimeric DNA target, wherein n₋₉ is g or n₊₉ is c, said variant has an arginine (R) or a lysine (K) in position 38. The I-*Cre*I the variants having the following amino acid residues in positions 28, 30, 33, 38 and 40 respectively, are examples of such variants : Q28/N30/Y33/K38/R40, R28/N30/K33/R38/Q40, Q28/N30/R33/R38/R40, Q28/N30/Y33/K38/K40, K28/N30/T33/R38/Q40, K28/N30/S33/R38/E40, S28/N30/Y33/R38/K40, K28/N30/S33/R38/D40, K28/N30/S33/R38/S40, Q28/N30/Y33/R38/K40, Q28/N30/K33lR38/T40, N28/N30/S33/R38/K40, N28/N30/S33/R38/R40, E28/N30/R33/R38/K40, R28/N30/T33/R38/A40, Q28/N30/Y33/R38/A40, Q28/N30/Y33/R38/S40, K28/N30/R33/K38/A40, R28/N30/A33/K38/S40, A28/N30/N33/R38/K40, Q28/N30/S33/R38/K40, K28/A30/H33/R38/S40, K28/H30/H33/R38/S40, K28/E30/S33/R38/S40, K28/N30/H33/R38/S40, K28/D30/H33/K38/S40, K28/K30/H33/R38/S40, K28/S30/H33/R38/S40, and K28/G30/V33/R38/S40.

More preferably, said DNA target comprises a nucleotide triplet in positions -10 to -8, which is selected from the group consisting of: aac, aag, aat, acc, acg, act, aga, agc, agg, agt, ata, atg, cag, cga, cgg, ctg, gac, gag, gat, gaa, gcc, gga, ggc, ggg, ggt, gta, gtg, gtt, tac, tag, tat, taa, tcc, tga, tgc, tgg, tgt or ttg, and/or a nucleotide triplet in positions +8 to +10, which is the reverse complementary sequence of said nucleotide triplet in positions -10 to -8.

In a second preferred embodiment of said variant, it is an I-*Mso*I variant having at least two substitutions, one in each of the two subdomains situated from positions 30 to 43 and 47 to 75 of I*-Mso*I*,* respectively.

Furthermore, other residues may be mutated on the entire sequence of the parent LAGLIDADG homing endonuclease, and in particular in the C-terminal half of said sequence. For example, the substitutions in the C-terminal half of I-*Cre*I (positions 80 to 163) are preferably in positions: 80, 82, 85, 86, 87, 94, 96, 100, 103, 114, 115, 117, 125, 129, 131, 132, 147, 151, 153, 154, 155, 157, 159 and 160 of I-*Cre*I*.*

The variants of the invention may include one or more residues inserted at the NH₂ terminus and/or COOH terminus of the parent LAGLIDADG homing endonuclease sequence. For example, a methionine residue is introduced at the NH₂ terminus, a tag (epitope or polyhistidine sequence) is introduced at the NH₂ terminus and/or COOH terminus; said tag is useful for the detection and/or the purification of said polypeptide.

The variants of the invention may be, either a monomer or single-chain chimeric endonuclease comprising two LAGLIDADG homing endonuclease domains in a single polypeptide, or an homodimer or heterodimer comprising two such domains in two separate polypeptides. According to the invention, one or both monomer(s)/domain(s) may be mutated in the two subdomains as defined above. One monomer/domain may be from a parent LAGLIDADG homing endonuclease or a functional variant thereof.

According to another preferred embodiment of the invention, said variant is a monomer, a single-chain chimeric molecule or an heterodimer, wherein both LAGLIDADG homing endonuclease domains comprise mutations in at least two separate subdomains, as defined above, said mutations in one domain being different from that in the other domain.

The subject-matter of the present invention is also a polynucleotide fragment encoding a variant or a mutated domain thereof, as defined above ; said polynucleotide may encode one domain of a monomer, one monomer of an homodimer or heterodimer, or two domains of a monomer or single-chain molecule, as defined above.

The subject-matter of the present invention is also a recombinant vector comprising at least one polynucleotide fragment encoding a variant, as defined above. Said vector may comprise a polynucleotide fragment encoding the monomer of a homodimeric variant or the two domains of a monomeric variant or a single-chain molecule. Alternatively, said vector may comprise two different polynucleotide fragments, each encoding one of the monomers of an heterodimeric variant.

One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors".

A vector according to the present invention comprises, but is not limited to, a YAC (yeast artificial chromosome), a BAC (bacterial artificial), a baculovirus vector, a phage, a phagemid, a cosmid, a viral vector, a plasmid, a RNA vector or a linear or circular DNA or RNA molecule which may consist of chromosomal, non chromosomal, semi-synthetic or synthetic DNA. In general, expression vectors of utility in recombinant DNA techniques are often in the form of "plasmids" which refer generally to circular double-stranded DNA loops which, in their vector form are not bound to the chromosome. Large numbers of suitable vectors are known to those of skill in the art.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), paramyxovirus (e. g. measles and Sendai), positive strand RNA viruses such as picornavirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example.

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture ; TRPI for *S*. *cerevisiae;* tetracycline, rifampicin or ampicillin resistance in *E*. *coli.*

Preferably said vectors are expression vectors, wherein the sequence(s) encoding the variant of the invention is placed under control of appropriate transcriptional and translational control elements to permit production or synthesis of said variant. Therefore, said polynucleotide is comprised in expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said variant is an heterodimer, the two polynucleotides encoding each of the monomers are included in one vector which is able to drive the expression of both polynucleotides, simultaneously.

According to another advantageous embodiment of said vector, it includes a targeting construct comprising sequences sharing homologies with the region surrounding the chimeric DNA target sequence as defined above.

More preferably, said targeting DNA construct comprises:
a) sequences sharing homologies with the region surrounding the chimeric DNA target sequence as defined above, and
b) sequences to be introduced flanked by sequence as in a).

The invention also concerns a prokaryotic or eukaryotic host cell which is modified by a polynucleotide or a vector as defined above, preferably an expression vector.

The invention also concerns a non-human transgenic animal or a transgenic plant, characterized in that all or part of their cells are modified by a polynucleotide or a vector as defined above.

As used herein, a cell refers to a prokaryotic cell, such as a bacterial cell, or eukaryotic cell, such as an animal, plant or yeast cell.

The polynucleotide sequence(s) encoding the variant as defined in the present invention may be prepared by any method known by the man skilled in the art. For example, they are amplified from a cDNA template, by polymerase chain reaction with specific primers. Preferably the codons of said cDNA are chosen to favour the expression of said protein in the desired expression system.

The recombinant vector comprising said polynucleotides may be obtained and introduced in a host cell by the well-known recombinant DNA and genetic engineering techniques.

The variant of the invention is produced by expressing the polypeptide(s) as defined above; preferably said polypeptide(s) are expressed or coexpressed in a host cell modified by one or two expression vector(s), under conditions suitable for the expression or co-expression of the polypeptides, and the variant is recovered from the host cell culture.

The subject-matter of the present invention is further the use of a variant, one or two polynucleotide(s), preferably included in expression vector(s), a cell, a transgenic plant, a non-human transgenic mammal, as defined above, for molecular biology, for *in vivo* or *in vitro* genetic engineering, and for *in vivo* or *in vitro* genome engineering, for non-therapeutic purposes.

Non therapeutic purposes include for example (i) gene targeting of specific loci in cell packaging lines for protein production, (ii) gene targeting of specific loci in crop plants, for strain improvements and metabolic engineering, (iii) targeted recombination for the removal of markers in genetically modified crop plants, (iv) targeted recombination for the removal of markers in genetically modified microorganism strains (for antibiotic production for example).

According to an advantageous embodiment of said use, it is for inducing a double-strand break in a site of interest comprising a chimeric DNA target sequence, thereby inducing a DNA recombination event, a DNA loss or cell death.

According to the invention, said double-strand break is for: repairing a specific sequence, modifying a specific sequence, restoring a functional gene in place of a mutated one, attenuating or activating an endogenous gene of interest, introducing a mutation into a site of interest, introducing an exogenous gene or a part thereof, inactivating or detecting an endogenous gene or a part thereof, translocating a chromosomal arm, or leaving the DNA unrepaired and degraded.

According to another advantageous embodiment of said use, said variant, polynucleotide(s), vector, cell, transgenic plant or non-human transgenic mammal are associated with a targeting DNA construct as defined above.

The subject-matter of the present invention is also a method of genetic engineering, characterized in that it comprises a step of double-strand nucleic acid breaking in a site of interest located on a vector comprising a chimeric DNA target as defined hereabove, by contacting said vector with a variant as defined above, thereby inducing a homologous recombination with another vector presenting homology with the sequence surrounding the cleavage site of said variant.

The subjet-matter of the present invention is also a method of genome engineering, characterized in that it comprises the following steps: 1) double-strand breaking a genomic locus comprising at least one chimeric DNA target of a variant as defined above, by contacting said target with said variant; 2) maintaining said broken genomic locus under conditions appropriate for homologous recombination with a targeting DNA construct comprising the sequence to be introduced in said locus, flanked by sequences sharing homologies with the targeted locus.

The subject-matter of the present invention is also a method of genome engineering, characterized in that it comprises the following steps: 1) double-strand breaking a genomic locus comprising at least one chimeric DNA target of a variant as defined above, by contacting said cleavage site with said variant; 2) maintaining said broken genomic locus under conditions appropriate for homologous recombination with chromosomal DNA sharing homologies to regions surrounding the cleavage site.

The subject-matter of the present invention is also a composition characterized in that it comprises at least one variant, one or two polynucleotide(s), preferably included in expression vector(s), as defined above.

In a preferred embodiment of said composition, it comprises a targeting DNA construct comprising the sequence which repairs the site of interest flanked by sequences sharing homologies with the targeted locus.

The subject-matter of the present invention is also the use of at least one variant, one or two polynucleotide(s), preferably included in expression vector(s), as defined above, for the preparation of a medicament for preventing, improving or curing a genetic disease in an individual in need thereof, said medicament being administrated by any means to said individual.

The subject-matter of the present invention is also a method for preventing, improving or curing a genetic disease in an individual in need thereof, said method comprising at least the step of administering to said individual a composition as defined above, by any means.

The subject-matter of the present invention is also the use of at least one variant, one or or two polynucleotide(s), preferably included in expression vector(s), as defined above for the preparation of a medicament for preventing, improving or curing a disease caused by an infectious agent that presents a DNA intermediate, in an individual in need thereof, said medicament being administrated by any means to said individual.

The subject-matter of the present invention is also a method for preventing, improving or curing a disease caused by an infectious agent that presents a DNA intermediate, in an individual in need thereof, said method comprising at least the step of administering to said individual a composition as defined above, by any means.

The subject-matter of the present invention is also the use of at least one variant, one or two polynucleotide(s), preferably included in expression vector(s), as defined above, *in vitro,* for inhibiting the propagation, inactivating or deleting an infectious agent that presents a DNA intermediate, in biological derived products or products intended for biological uses or for disinfecting an object.

The subject matter of the present invention is also a method for decontaminating a product or a material from an infectious agent that presents a DNA intermediate, said method comprising at least the step of contacting a biological derived product, a product intended for biological use or an object, with a composition as defined above, for a time sufficient to inhibit the propagation, inactivate or delete said infectious agent.

In a particular embodiment, said infectious agent is a virus. For example said virus is an adenovirus (Ad11, Ad21), herpesvirus (HSV, VZV, EBV, CMV, herpesvirus 6, 7 or 8), hepadnavirus (HBV), papovavirus (HPV), poxvirus or retrovirus (HTLV, HIV).

The subject-matter of the present invention is also the use of at least one homing endonuclease variant, as defined above, as a scaffold for making other meganucleases. For example a third round of mutagenesis and selection/screening can be performed on said variants, for the purpose of making novel, third generation homing endonucleases.

According to another advantageous embodiment of said uses, said homing endonuclease variant is associated with a targeting DNA construct as defined above.

The use of the homing endonuclease variant and the methods of using said homing endonuclease variant according to the present invention include also the use of the single-chain chimeric endonuclease derived from said variant, the polynucleotide(s), vector, cell, transgenic plant or non-human transgenic mammal encoding said variant or single-chain chimeric endonuclease, as defined above.

In addition to the preceding features, the invention further comprises other features which will emerge from the description which follows, which refers to examples illustrating the I-*Cre*I meganuclease variants and their uses according to the invention, as well as to the appended drawings in which:
- figure 1 illustrates the principle of the invention. **A**: Structure of I-*Cre*I bound to its target. Experimental data have shown that two independent subdomains (squares) could be identified in the DNA binding domain; each subdomain of the core domain binds a different half of the DNA target. **B**. One would like to identify smaller independent subdomains (squares), each binding a distinct part of a half DNA target. However, there is no structural or experimental data in favour of this hypothesis,
- figure 2 represents the map of the base specific interactions of I-*Cre*I with its DNA target, after Chevalier and Stoddard, Nucleic Acids Res., 2001, 29, 3757-74 ; Chevalier et al. J. Mol. Biol., 2003, 329, 253-69. The inventor has identified novel I-*Cre*I derived endonucleases able to bind DNA targets modified in regions -10 to -8 and 8 to 10, or -5 to -3 and 3 to 5. These DNA regions are indicated in grey boxes.
- figure 3 illustrates the strategy for the making of redesigned Homing Endonucleases. **a**. General strategy. A large collection of I-*Cre*I derivatives with locally altered specificity is generated. Then, a combinatorial approach is used to assemble these mutants into homodimeric proteins, and then into heterodimers, resulting in a meganucleases with fully redesigned specificity. **b**. Making of combinatorial mutants cleaving the COMB1 target (SEQ ID NO: 53): a workflow. Two palindromic targets (COMB2 (SEQ ID NO: 39)) and COMB3 (SEQ ID NO: 46)) are derived from the COMB1 target, and homodimeric combinatorial mutants are designed to cleave these two targets. Positives are then coexpressed to cleave the COMB1 target **c**. The RAG1 series of target. Two palindromic targets (RAG1.2 (SEQ ID NO: 55) and RAG1.3 (SEQ ID NO: 56)) are derived from RAG1.1 (SEQ ID NO: 54)). Then, a worflow similar to that described for the COMB series of target can be applied.

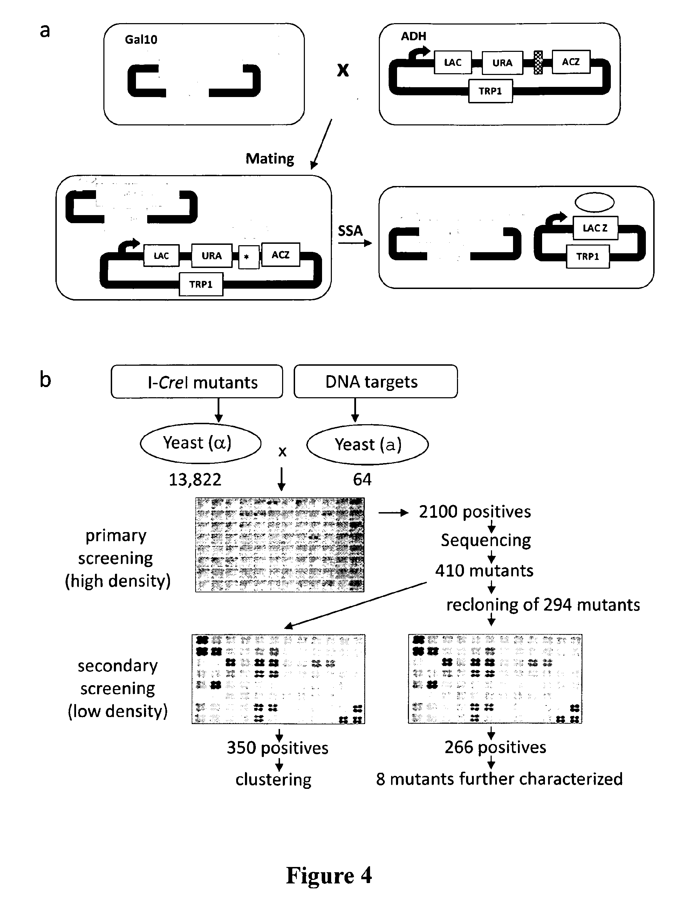

- figure 4 illustrates the screening of the variants. (a) Yeast screening assay principle. A strain expressing the meganuclease to be assayed (MEGA), marked with the *LEU2* gene, is mated with a strain harboring a reporter plasmid containing the chosen target, marked with the *TRP1* gene. The target is flanked by overlapping truncated LacZ genes (LAC and ACZ). In diploids (*LEU2, TRP1*), cleavage of the target site by the meganuclease induces homologous recombination between the two LacZ repeats, resulting in a functional beta-galactosidase gene, that can be monitored by X-gal staining. (b) Scheme of an experiment. A library of I-*Cre*I variants is built using PCR, cloned into a replicative yeast expression vector and transformed in *S*. *cerevisiae* strain FYC2-6A (*MATα, trp1*Δ*63, leu2*Δ*1, his3*Δ*200*). The 64 palindromic targets are cloned in the LacZ-based yeast reporter vector, and the resulting clones transformed into strain FYBL2-7B (*MATa, ura3*Δ*851, trp1Δ63, leu2Δ1, lys2Δ202*)*.* Robot-assisted gridding on filter membrane is used to perform mating between individual clones expressing meganuclease variants and individual clones harboring a reporter plasmid. After primary high throughput screening, the ORF of positive clones are amplified by PCR and sequenced. 410 different variants at positions 44, 68 and 70, derived from the I*-Cre*I N75 scaffold protein, were identified among the 2100 positives, and tested at low density, to establish complete patterns, and 350 clones were validated. Also, 294 mutants were recloned in yeast vectors, and tested in a secondary screen, and results confirmed those obtained without recloning. Chosen clones are then assayed for cleavage activity in a similar CHO-based assay and eventually *in vitro.*

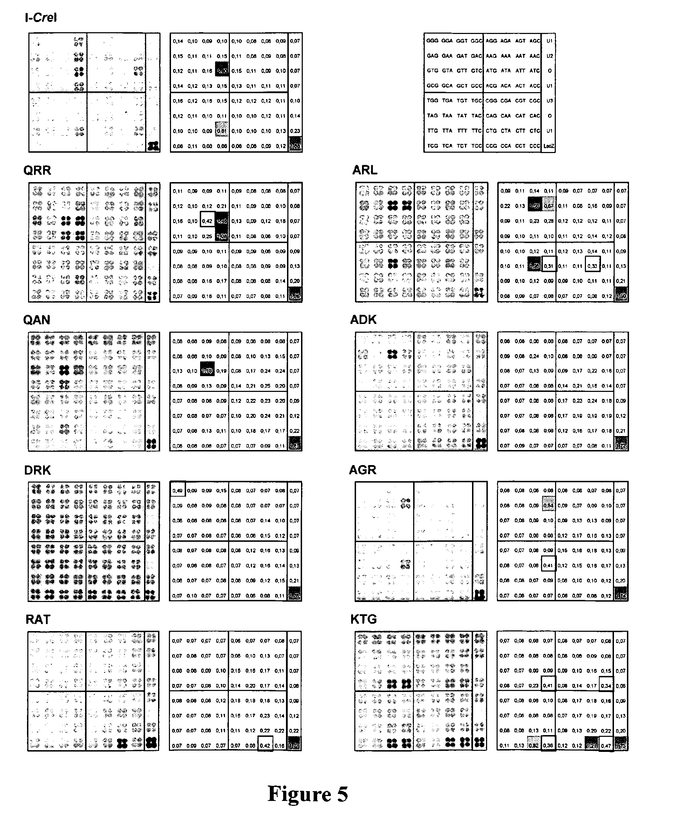

- figure 5 illustrates the cleavage patterns of a series of variants. Mutants are identified by three letters, corresponding to the residues in positions 44, 68 and 70. Each mutant is tested versus the 64 targets derived from the C1221 palindromic target cleaved by I*-Cre*I, by substitution of the nucleotides in positions ± 3 to 5, and a series of control targets. Target map is indicated in the top right panel. Cleavage patterns in yeast (left) and mammalian cells (right) for the I-*Cre*I protein, and 8 derivatives. For yeast, the initial raw data (filter) is shown. For CHO cells, quantitative raw data (ONPG measurement) are shown, values superior to 0.25 are boxed, values superior to 0.5 are highlighted in medium grey, values superior to 1 in dark grey. LacZ: positive control. 0: no target. U1, U2 and U3: three different uncleaved controls.

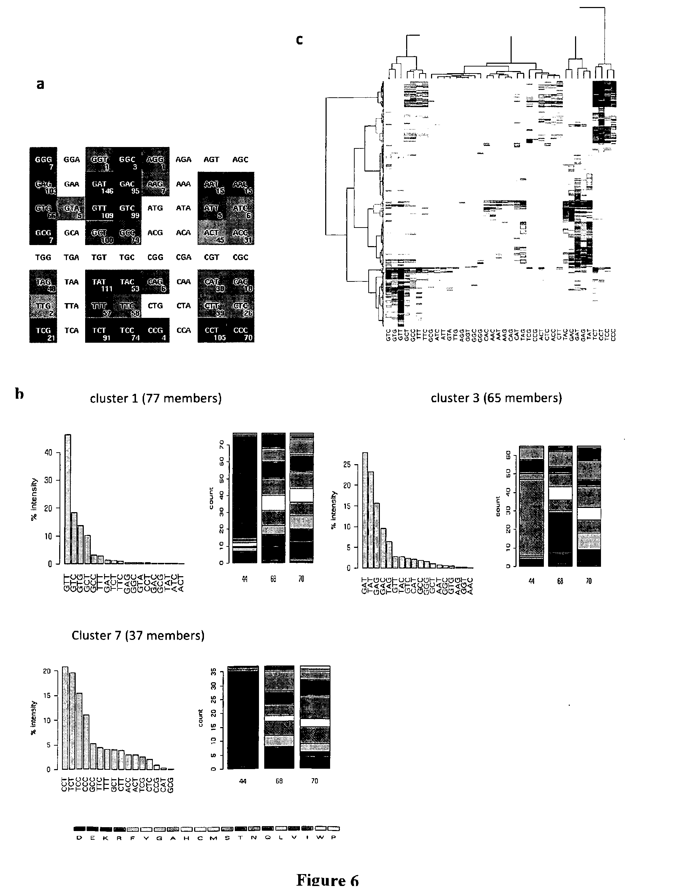

- figure 6 represents the statistical analysis. (a) Cleaved targets:
   targets cleaved by I-*Cre*I variants are colored in grey. The number of proteins cleaving each target is shown below, and the level of grey coloration is proportional to the average signal intensity obtained with these cutters in yeast. (b) Analysis of 3 out of the 7 clusters. For each mutant cluster (clusters 1, 3 and 7), the cumulated intensities for each target was computed and a bar plot (left column) shows in decreasing order the normalized intensities. For each cluster, the number of amino acid of each type at each position (44, 68 and 70) is shown as a coded histogram in the right column. The legend of amino-acid color code is at the bottom of the figure. (c) Hierarchical clustering of mutant and target data in yeast. Both mutants and targets were clustered using hierarchical clustering with Euclidean distance and Ward's method (Ward, J.H., American statist. Assoc., 1963, 58, 236-244). Clustering was done with hclust from the R package. Mutants and targets dendrograms were reordered to optimize positions of the clusters and the mutant dendrogram was cut at the height of 8 with deduced clusters. QRR mutant and GTC target are indicated by an arrow. Gray levels reflects the intensity of the signal.

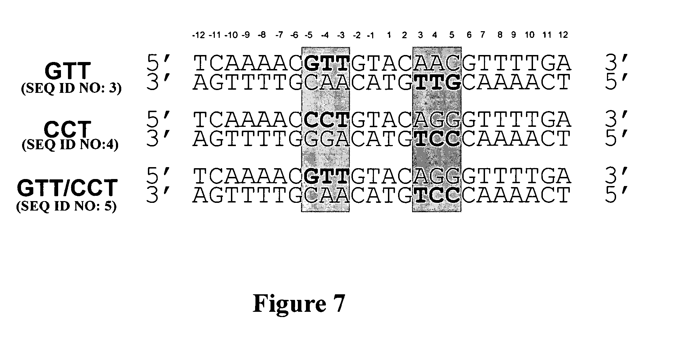

- figure 7 illustrates an example of hybrid or chimeric site: gtt (SEQ ID NO: 3) and cct (SEQ ID NO: 4) are two palindromic sites derived from the I-*Cre*I site. The gtt/cct hybrid site (SEQ ID NO: 5) displays the gtt sequence on the top strand in -5, -4, -3 and the cct sequence on the bottom strand in 5, 4, 3.

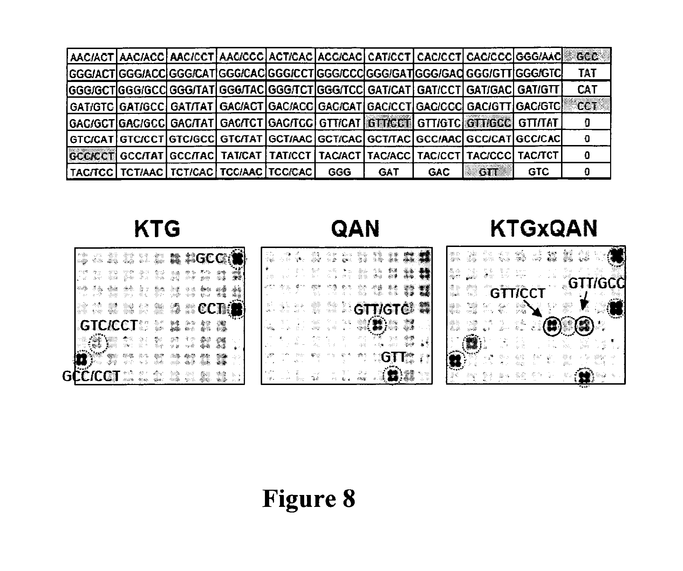

- figure 8 illustrates the cleavage activity of the heterodimeric variants. Yeast were co-transformed with the KTG and QAN variants. Target organization is shown on the top panel: target with a single gtt, cct or gcc half site are in bold; targets with two such half sites, which are expected to be cleaved by homo- and/or heterodimers, are in bold and highlighted in grey ; 0: no target. Results are shown on the three panels below. Unexpected faint signals are observed only for gtc/cct and gtt/gtc, cleaved by KTG and QAN, respectively.

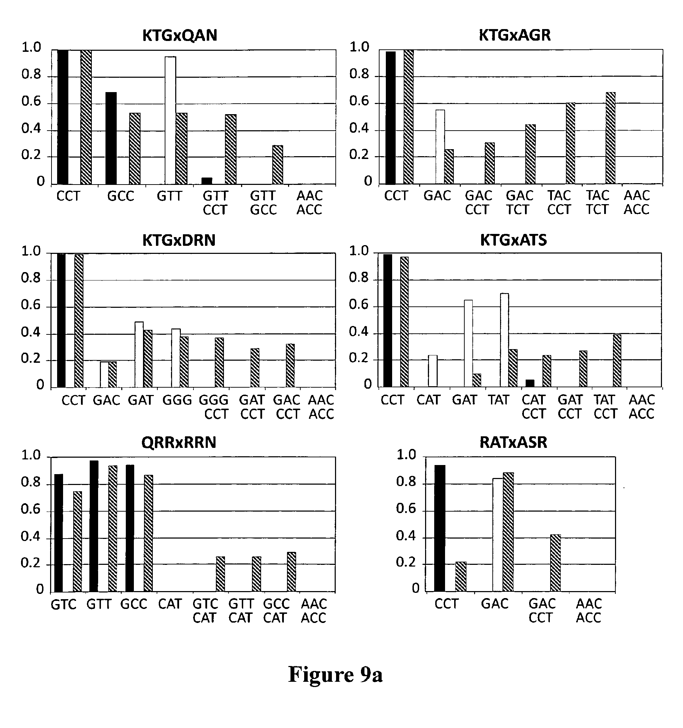

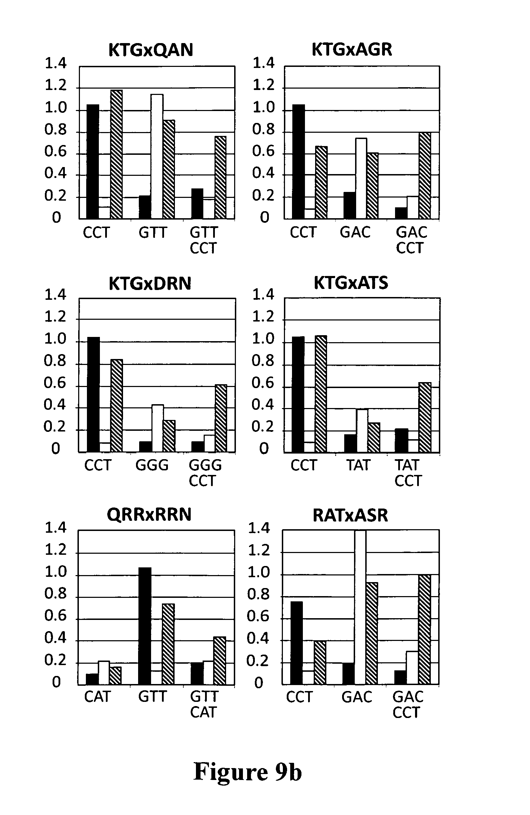

- figure 9 represents the quantitative analysis of the cleavage activity of the heterodimeric variants. (a) Co-transformation of selected mutants in yeast. For clarity, only results on relevant hybrid targets are shown. The aac/acc target is always shown as an example of unrelated target. For the KTGxAGR couple, the palindromic tac and tct targets, although not shown, are cleaved by AGR and KTG, respectively. Cleavage of the cat target by the RRN mutant is very low, and could not be quantified in yeast. (b) Transient co-transfection in CHO cells. For (a) and (b), Black bars: signal for the first mutant alone; grey bars: signal for the second mutant alone; striped bars: signal obtained by co-expression or cotransfection.

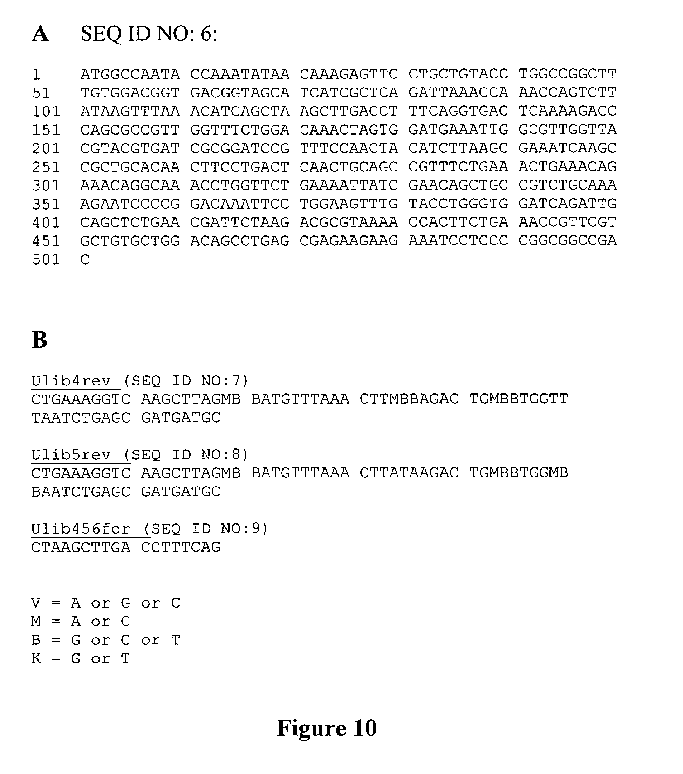

- figure 10 represents the sequences of the I*-Cre*I N75 scaffold protein and degenerated primers used for the Ulib4 and Ulib5 libraries construction. A. The scaffolf (SEQ ID NO: 6) is the I*-Cre*I ORF including the D75N codon substitution and three additional codons (AAD) at the 3' end. B. Primers (SEQ ID NO: 7, 8, 9),

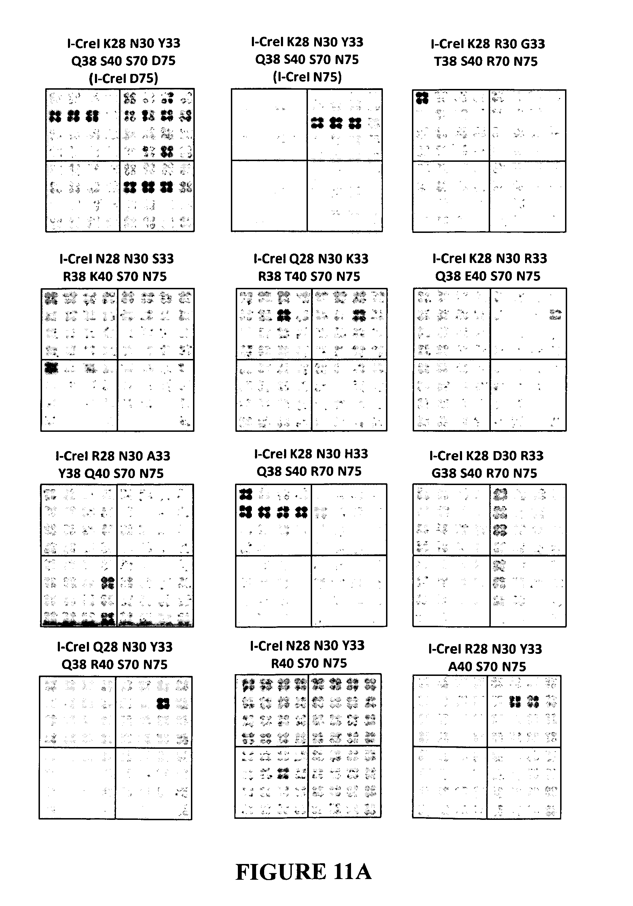

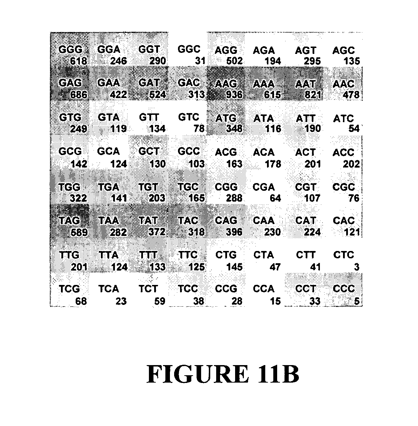

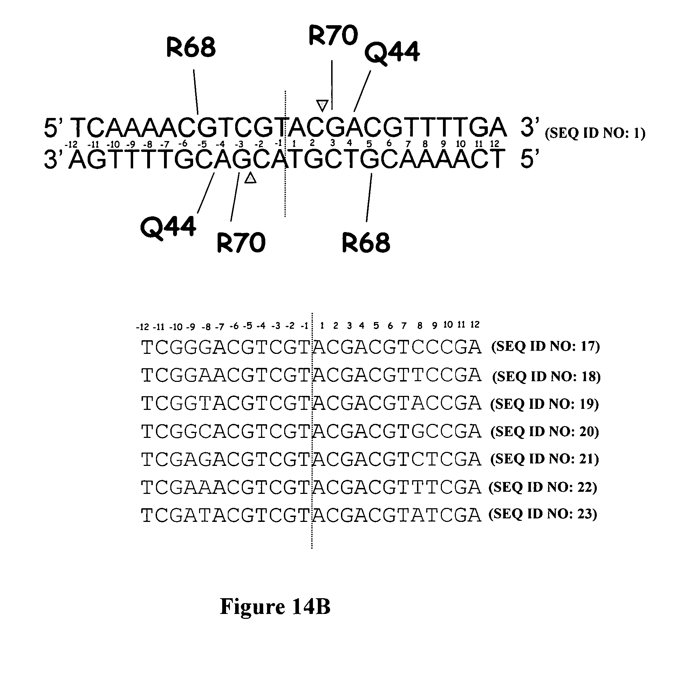

- figure 11 illustrates examples of patterns and the numbers of mutants cleaving each target. A. Examples of profiling. Each novel endonuclease is profiled in yeast on a series of 64 palindromic targets, arrayed as in figure 11B, differing from the sequence shown in figure 2, at positions ±8, ±9 and ±10. Each target sequence is named after the -10,-9,-8 triplet (10NNN). For example GGG corresponds to the tcgggacgtcgtacgacgtcccga target (SEQ ID NO:17; figure 14B). Meganucleases are tested 4 times against the 64 targets. Targets cleaved by I*-Cre*I (D75), I*-Cre*I N75 or ten derived variants are visualised by black or grey spots. B. Numbers of mutants cleaving each target, and average intensity of cleavage. Each sequence is named after the -10,-9,-8 triplet (10NNN). The number of proteins cleaving each target is shown below, and the level of grey coloration is proportional to the average signal intensity obtained with these cutters in yeast.

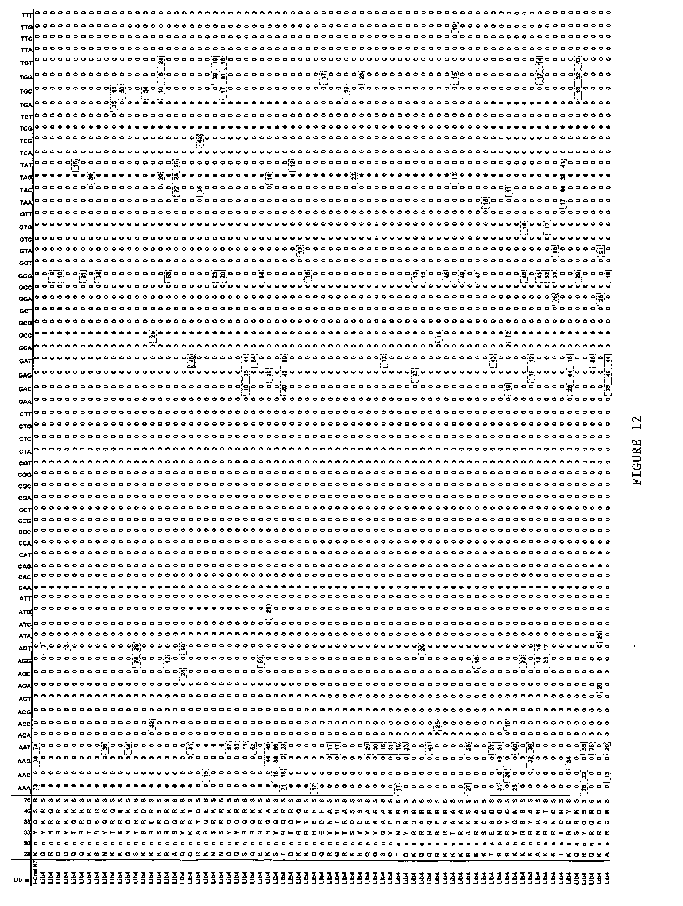

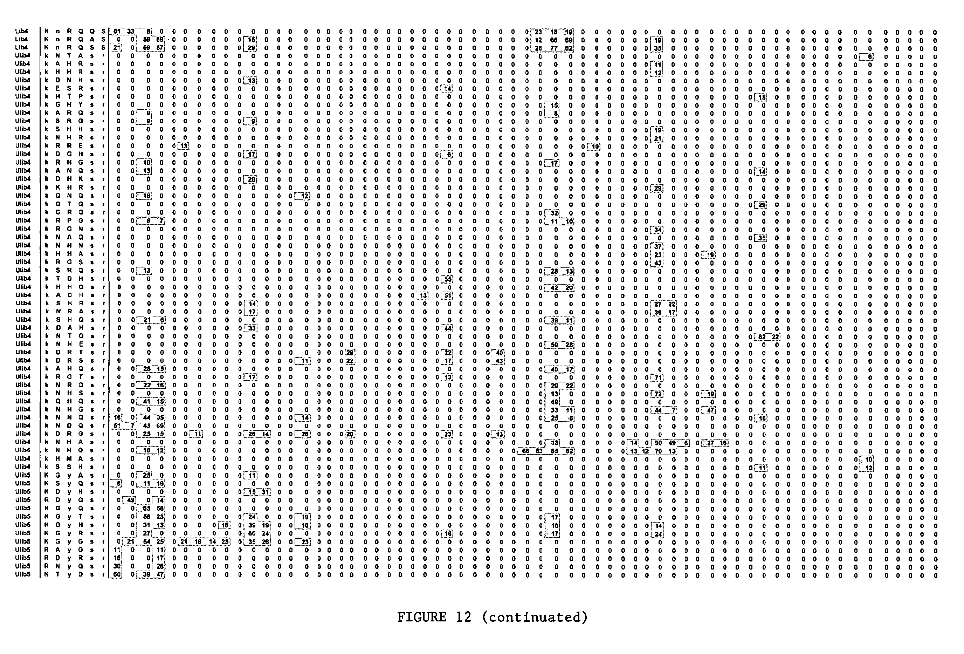

- figure 12 represents the cleavage patterns of the I-*Cre*I variants in position 28, 30, 33, 38 and/or 40. For each of the 141 I*-Cre*I variants obtained after screening, and defined by residues in position 28, 30, 33, 38, 40, 70 and 75, cleavage was monitored in yeast with the 64 targets derived from the C 1221 palindromic target cleaved by I*-Cre*I, by substitution of the nucleotides in positions ± 8 to 10.Targets are designated by three letters, corresponding to the nucleotides in position -10, -9 and -8. For example GGG corresponds to the tcgggacgtcgtacgacgtcccga target (SEQ ID NO: 17). Values (boxed) correspond to the intensity of the cleavage, evaluated by an appropriate software after scanning of the filter, whereas (0) indicates no cleavage.

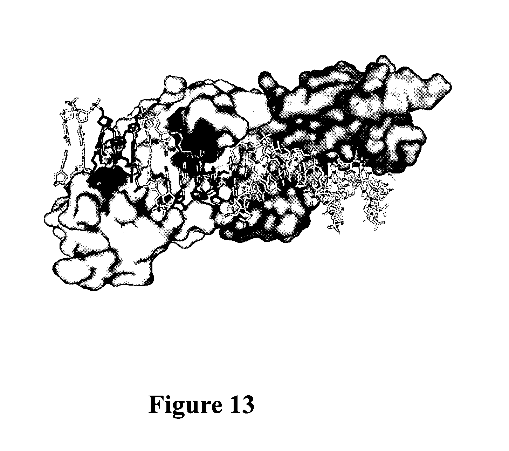

- figure 13 represents the localisation of the mutations in the protein and DNA target, on a *I-CreI* homodimer bound to its target. The two set of mutations (residues 44, 68 and 70; residues 30, 33 and 38) are shown in black on the monomer on the left. The two sets of mutations are clearly distinct spatially. However, there is no structural evidence for distinct subdomains. Cognate regions in the DNA target site (region -5 to -3; region -10 to -8) are shown in grey on one half site.

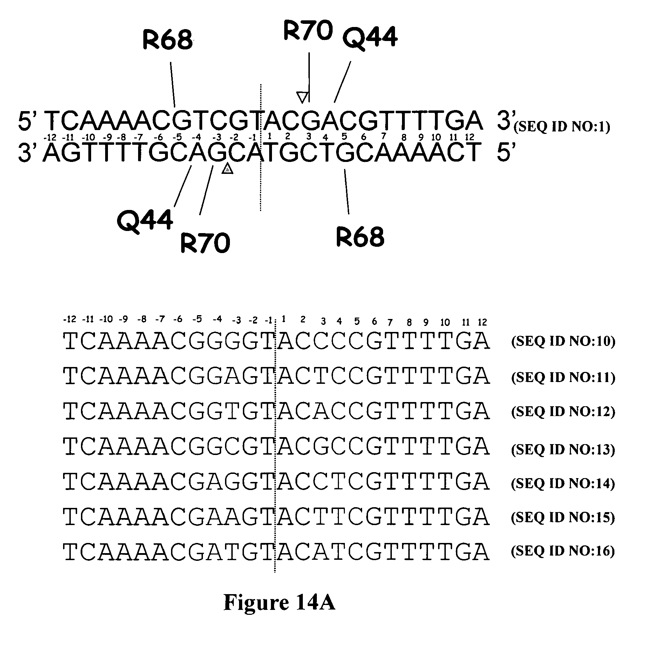

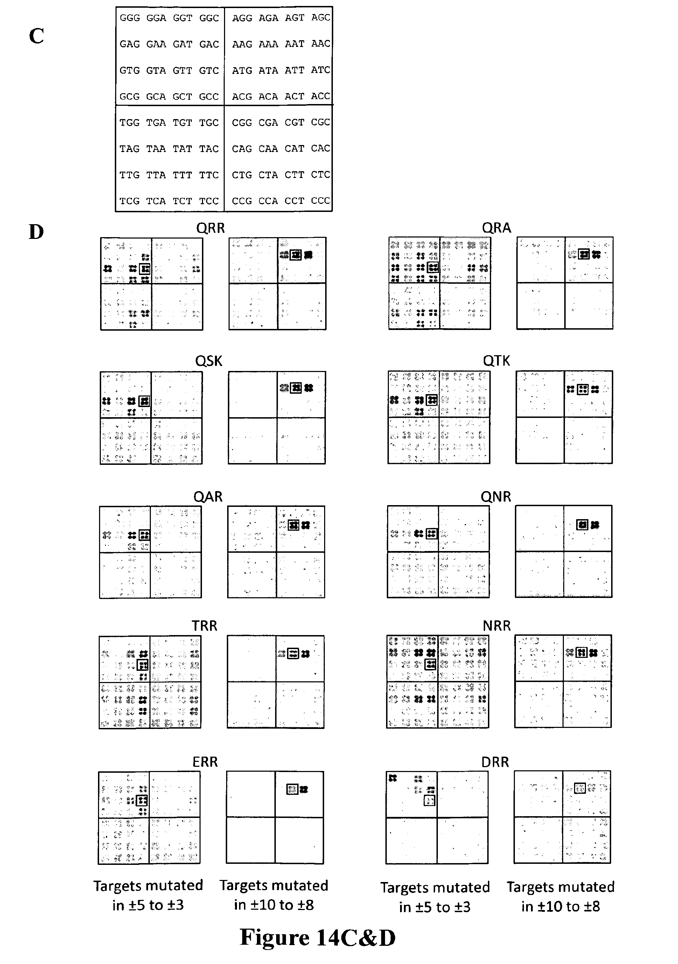

- figure 14: I-*Cre*I derivative target definition (A and B) and profiling (C and D). All targets are derived from C1221, a palindromic target cleaved by I*-Cre*I wild-type, and shown on the top of A and B. **A**. A first series of 64 targets is derived by mutagenesis of positions ±5 to ±3 (in grey boxes). A few examples are shown below. Interactions with I-*Cre*I residues 44, 68 and 70 are shown. **B**. A second series of 64 target is derived by mutagenesis of positions ±10 to ±8 (in grey boxes). A few examples are shown below. Positions ±8, ±9 and ±10 are not contacted by residues 44, 68 and 70. **C**. Organisation of the targets as in Figure 13D. For the left panel, the three letters in the table indicate the bases in positions ±3, ±4 and ±5 (for example, GGG means tcaaaac**ggg**gta**ccc**cgttttga (SEQ ID NO: 10)). For the right panels, the three letters indicate the bases in positions ±8, ±9 and ±10 (for example, GGG means tc**ggg**acgtcgtacgacgt**ccc**ga (SEQ ID NO: 17)). **D**. Profiling. Ten I*-Cre*I variants cleaving the C1221 target, including I-CreI N75 (QRR) are profiled with the two sets of 64 targets (±5 to ±3 on the left, and ±10 to ±8 on the right). Targets are arranged as in Figure 13C. The C1221 target (squared) is found in both sets. Mutants are identified by three letters corresponding to the residues found in position 44, 68 and 70 (example:QRR is Q44, R68, R70), and all of them have an additional D75N mutation.

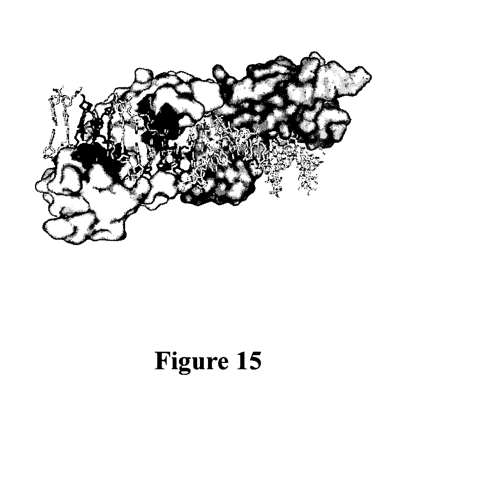

- figure 15 represents the localisation of the mutations in the protein and DNA target, on a I-*Cre*I homodimer bound to its target. The two set of mutations (residues 44, 68 and 70; residues 28, 30, 33, 38 and 40 are shown in black on the monomer on the left. The two sets of mutations are clearly distinct spatially. However, there is no structural evidence for distinct subdomains. Cognate regions in the DNA target site (region -5 to -3; region -10 to -8) are shown in grey on one half site.

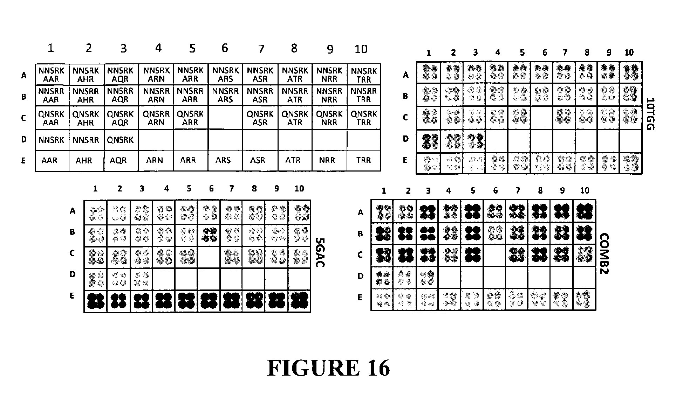

- figure 16 illustrates combination of mutations in positions 44, 68, 70 and 28, 30, 33, 38, 40, to cleave the chimeric target COMB2 (tc**tgg**acg**a**cgtacg**t**cgt**cct**ga: SEQ ID NO: 39). Top panel: map of the mutants feature on the following panels. As described in text, combinatorial mutants are named with a eight letter code, after residues at positions 28, 30, 33, 38, 40, 44, 68 and 70 and parental controls with a five letter or three letter code, after residues at positions 28, 30, 33, 38 and 40 or 44, 68 and 70. Mutants are screened in yeast against COMB2 and 10TGC and 5GAC, the two parental targets.

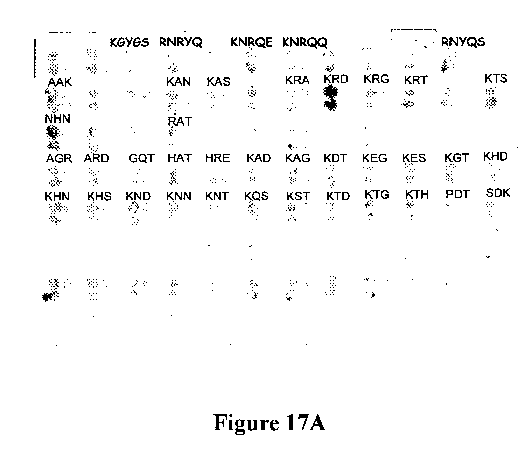

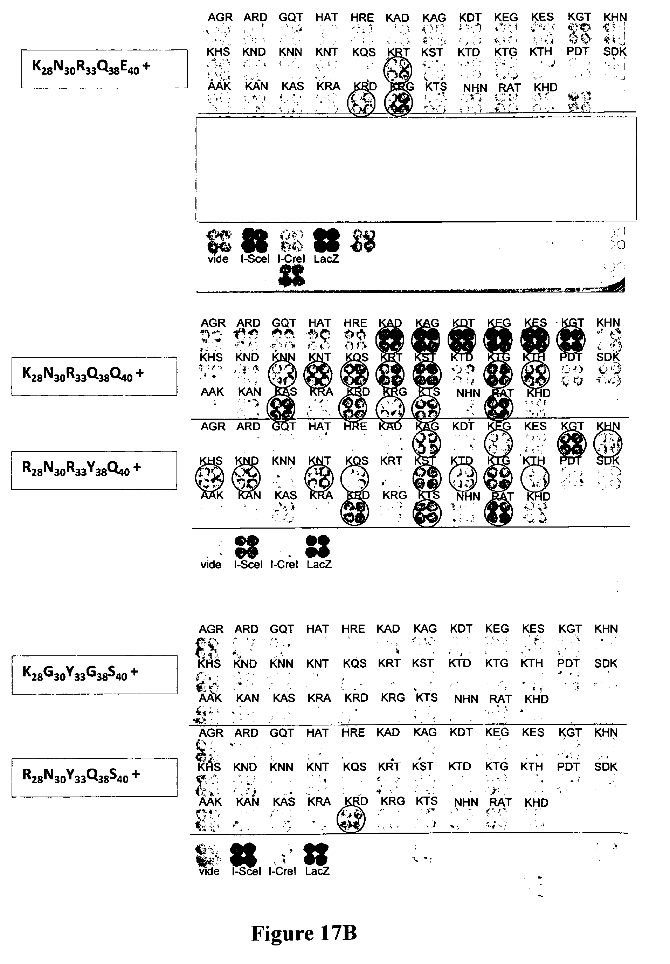

- figure 17 illustrates combination of mutations in positions 44, 68, 70 and 28, 30, 33, 38, 40, to cleave the chimeric tc**aa**cac**cct**gtac**ag**ggt**gtt**ga target (SEQ ID NO:49). A. Proteins mutated either in 44, 68 and 70, either on 28, 30, 33, 38 and 40, are assayed on the chimeric target. Proteins mutated in 44, 68 and 70 are called with a three letters code, indicating the amino acid residues in positions 44, 68 and 70 (example: AAK means A44, A68, K70). Proteins mutated in 28, 30, 33, 38 and 40 are called with a five letters code, indicating the amino acid residues in positions 28, 30, 33, 38 and 40 (example: KNRQQ means K28, N30, R33, Q38, Q40). B. Chimeric proteins are assayed on the chimeric DNA target. Proteins are defined by the mutations in 28, 30, 33, 38, 40, indicated on the left of the panel, and by the mutations in 44, 68 and 70, indicated by the three letters code on the panel. Chimeric proteins cleaving the chimeric DNA target are circled.

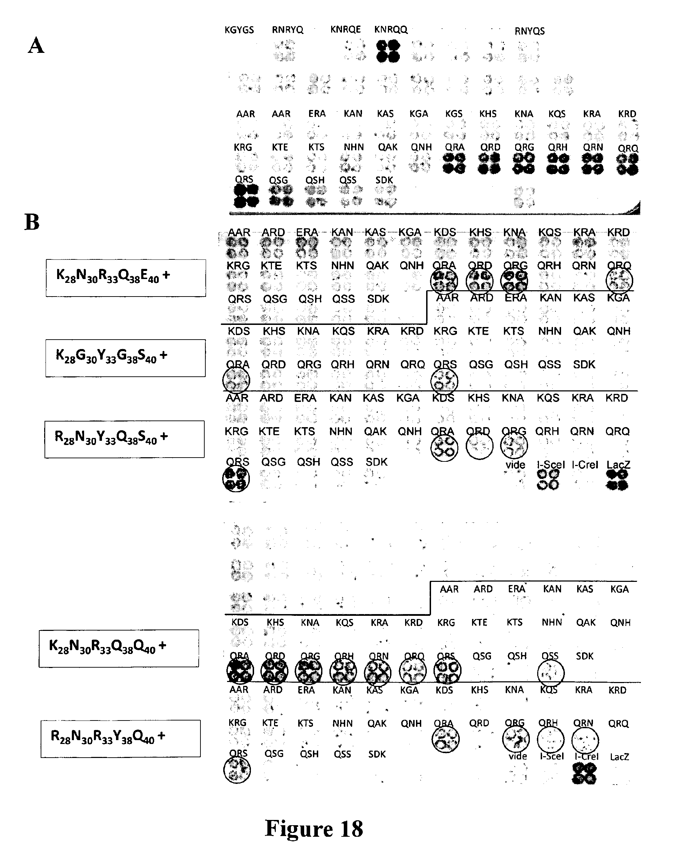

- figure 18 illustrates combination of mutations in positions 44, 68, 70 and 28, 30, 33, 38, 40, to cleave the chimeric tcaa**c**ac**t**t**t**gtac**a**a**a**g**tgt**tga target (SEQ ID NO:52). **A**. Proteins mutated either in 44, 68 and 70, either on 28, 30, 33, 38 and 40, are assayed on the chimeric target. Proteins mutated in 44, 68 and 70 are called with a three letters code, indicating the amino acid residues in positions 44, 68 and 70 (example: AAR means A44, A68, R70). Proteins mutated in 28, 30, 33, 38 and 40 are called with a five letters code, indicating the amino acid residues in positions 28, 30, 33, 38 and 40 (example: KNRQE means K28, N30, R33, Q38, E40). B. Chimeric proteins are assayed on the chimeric DNA target. Proteins are defined by the mutations in 28, 30, 33, 38, 40, indicated on the left of the panel, and by the mutations in 44, 68 and 70, indicated by the three letters code on the panel.

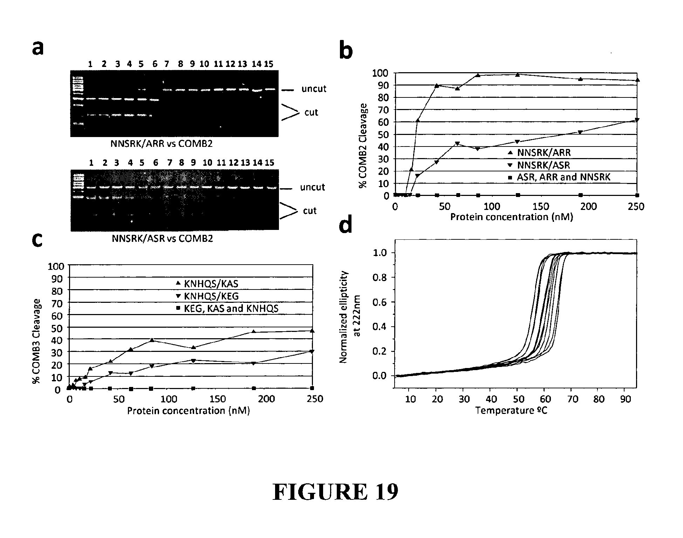

- figure 19 illustrates the biochemical and biophysical characterization of combinatorial mutants. **a**. Examples of raw data for *in vitro* cleavage. Different concentrations of proteins were assayed. Lanes 1 to 15: protein concentrations in nM are 250, 189.4, 126.3, 84.2, 63.2, 42.1, 21.1, 15.8, 10.5, 7.4,4.2, 2.1, 1.0, 0.5 and 0. **b**. Cleavage of COMB2 by combinatorial mutants. c. Cleavage of COMB3 by combinatorial mutants. **d**. Thermal denaturation of the same proteins measured by CD. The bold line corresponds to I-*Cre*I N75, with a mid point denaturation temperature of 65°C. Other proteins: KNHQS/KEG (mid point denaturation temperature: 65.3°C), KNHQS/KAS (64.9°C), KEG (63.1°C),KNHQS (62.2°C), NNSRQ (61.2°C), KAS (61.2°C), KAS (61.2°C), ARR (57.3°C), ASR (57.1°C), NNSRK/ARR (55.8°C), NNSRK/ASR (55.8°C). For protein nomenclature, see Figure 16.

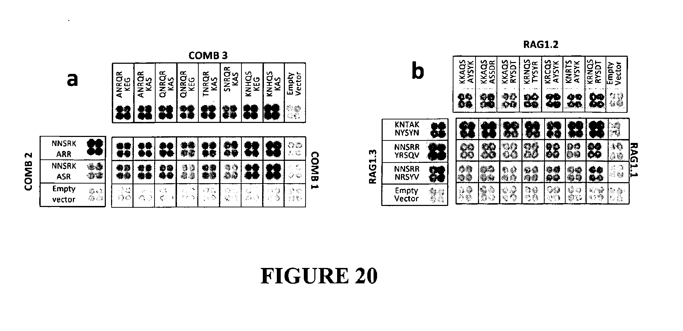

- figure 20 illustrates the cleavage of non palindromic target by redesigned heterodimers. **a**. Cleavage of COMB1 by heterodimers (bottom right panel). Cleavage of COMB2 and COMB3 palindromic targets by the parent homodimers is indicated on the top and left panel. For combinatorial mutants, nomenclature is the same as for Figure 16 and in text **b**. Cleavage of RAG1.1 target by heterodimers. As described in text, combinatorial mutants are named after 10 residues instead of 8, corresponding to positions 28, 30, 33, 38, 40, 44, 68, 70, 75 and 77.

### Example 1: Screening for new functional endonucleases: engineering of I-CreI variants with new specificity towards nucleotides ±3 to ±5 (5NNN)

The method for producing meganuclease variants and the assays based on cleavage-induced recombination in mammal or yeast cells, which are used for screening variants with altered specificity, are described in the International PCT Application WO 2004/067736 and Epinat et al., Nucleic Acids Res., 2003, 31, 2952-2962. These assays result in a functional LacZ reporter gene which can be monitored by standard methods (Figure 4a).

### A) Material and methods

### a) Construction of mutant libraries

I-*Cre*I scaffold-proteins open reading frames were synthesized, as described previously (Epinat et al., N.A.R., 2003, 31, 2952-2962). The I-*Cre*I scaffold proteins include wild-type I-*Cre*I, I-*Cre*I D75N (I-*Cre*I N75), I-*Cre*I R70S, D75N (I-*Cre*I S70 N75), I-*Cre*I I24V, R70S, D75N (I-*Cre*I V24 S70 N75), and I-*Cre*I I24V, R70S (I-*Cre*I V24 S70). Combinatorial libraries were derived from the I-*Cre*I scaffold proteins, by replacing different combinations of residues, potentially involved in the interactions with the bases in positions ± 3 to 5 of one DNA target half-site (Q44, R68, R70, D75 and I77). The diversity of the meganuclease libraries was generated by PCR using degenerated primers harboring a unique degenerated codon at each of the selected positions. For example, mutation D75N was introduced by replacing codon 75 with aac. Then, PCR on the I-*Cre*I N75 cDNA template was performed using primers from Sigma harboring codon VVK (18 codons, amino acids ADEGHKNPQRST) at positions 44, 68 and 70. The final PCR product was digested with specific restriction enzymes, and cloned back into the I-*Cre*I ORF digested with the same restriction enzymes, in pCLS0542. In this 2 micron-based replicative vector marked with the *LEU2* gene, I-*Cre*I variants are under the control of a galactose inducible promoter (Epinat et al., precited). After electroporation in *E. coli*, 7x10⁴ clones were obtained representing 12 times the theoretical diversity at the DNA level (18³ = 5832).

### b) Construction of target clones

The C1221 twenty-four bp palindrome (tcaaaacgtcgtacgacgttttga, SEQ ID NO: 1) is a repeat of the half-site of the nearly palindromic natural I-*Cre*I target (tcaaaacgtcgtgagacagtttgg, SEQ ID NO: 24). C1221 is cleaved as efficiently as the I-*Cre*I natural target *in vitro* and *ex vivo* in both yeast and mammalian cells. The 64 palindromic targets were derived from C1221 as follows: 64 pair of oligonucleotides (ggcatacaagtttcaaaacnnngtacnnngttttgacaatcgtctgtca (SEQ ID NO: 25) and reverse complementary sequences) were ordered form Sigma, annealed and cloned into pGEM-T Easy (PROMEGA) in the same orientation. Next, a 400 bp *Pvu*II fragment was excised and cloned into the yeast vector pFL39-ADH-LACURAZ, also called pCLS0042, and the mammalian vector pcDNA3.1-LACURAZ-ΔURA, both described previously (Epinat et al., 2003, precited), resulting in 64 yeast reporter vectors (target plasmids).

Alternatively, double-stranded target DNA, generated by PCR amplification of the single stranded oligonucleotides, was cloned using the Gateway protocol (INVITROGEN) into yeast and mammalian reporter vectors.

### c) Yeast strains

The library of meganuclease expression variants was transformed into the *leu2* mutant haploid yeast strain FYC2-6A: alpha, trp1Δ63, leu2Δ1, his3Δ200. A classical chemical/heat choc protocol derived from (Gietz and Woods, Methods Enzymol., 2002, 350, 87-96), that routinely gives 10⁶ independent transformants per µg of DNA, was used for transformation. Individual transformant (Leu⁺) clones were individually picked in 96 wells microplates.13824 colonies were picked using a colony picker (QpixII, GENETIX), and grown in 144 microtiter plates.

The 64 target plasmids were transformed using the same protocol, into the haploid yeast strain FYBL2-7B: a, ura3Δ851, trp1Δ63, leu2Δ1, lys2Δ202, resulting in 64 tester strains.

### d) Mating of meganuclease expressing clones and screening in yeast

Meganuclease expressing clones were mated with each of the 64 target strains, and diploids were tested for beta-galactosidase activity, by using the screening assay illustrated on figure 4. I-*Cre*I variant clones as well as yeast reporter strains were stocked in glycerol (20 %) and replicated in novel microplates. Mating was performed using a colony gridder (QpixII, GENETIX). Mutants were gridded on nylon filters covering YPD plates, using a high gridding density (about 20 spots/cm²). A second gridding process was performed on the same filters to spot a second layer consisting of 64 or 75 different reporter-harboring yeast strains for each variant. Membranes were placed on solid agar YPD rich medium, and incubated at 30 °C for one night, to allow mating. Next, filters were transferred to synthetic medium, lacking leucine and tryptophan, with galactose (2 %) as a carbon source (and with G418 for coexpression experiments), and incubated for five days at 37°C, to select for diploids carrying the expression and target vectors. After 5 days, filters were placed on solid agarose medium with 0.02 % X-Gal in 0.5 M sodium phosphate buffer, pH 7.0, 0.1 % SDS, 6 % dimethyl formamide (DMF), 7 mM β-mercaptoethanol, 1 % agarose, and incubated at 37 °C, to monitor β-galactosidase activity. After two days of incubation, positive clones were identified by scanning. The β-galactosidase activity of the clones was quantified using an appropriate software.

The clones showing an activity against at least one target were isolated (first screening). The spotting density was then reduced to 4 spots/cm² and each positive clone was tested against the 64 reporter strains in quadruplicate, thereby creating complete profiles (secondary screening).

### e) Sequence

The open reading frame (ORF) of positive clones identified during the primary and/or secondary screening in yeast was amplified by PCR on yeast colonies, by using the pair of primers: ggggacaagtttgtacaaaaaagcaggcttcgaaggagatagaaccatggccaataccaaatataacaaagagttcc (SEQ ID NO: 26) and ggggaccactttgtacaagaaagctgggtttagtcggccgccggggaggatttcttcttctcgc (SEQ ID NO: 27) from PROLIGO._Briefly, yeast colony is picked and resuspended in 100 µl of LGlu liquid medium and cultures overnight. After centrifugation, yeast pellet is resuspended in 10 µl of sterile water and used to perform PCR reaction in a final volume of 50 µl containing 1.5 µl of each specific primers (100 pmol/µl). The PCR conditions were one cycle of denaturation for 10 minutes at 94 °C, 35 cycles of denaturation for 30 s at 94°C, annealing for 1 min at 55°C, extension for 1.5 min at 72 °C, and a final extension for 5 min. The resulting PCR products were then sequenced.

### f) Re-Cloning of primary hits

The open reading frames (ORFs) of positive clones identified during the primary screening were recloned using the Gateway protocol (Invitrogen). ORFs were amplified by PCR on yeast colonies, as described in e). PCR products were then cloned in : (i) yeast gateway expression vector harboring a galactose inducible promoter, *LEU2* or KanR as selectable marker and a 2 micron origin of replication, and (ii) a pET 24d(+) vector from NOVAGEN. Resulting clones were verified by sequencing (MILLEGEN).

### B) Results

I-*Cre*I is a dimeric homing endonuclease that cleaves a 22 bp pseudo-palindromic target. Analysis of I-*Cre*I structure bound to its natural target has shown that in each monomer, eight residues establish direct interactions with seven bases (Jurica et al., 1998, precited). Residues Q44, R68, R70 contact three consecutive base pairs at position 3 to 5 (and -3 to -5, Figure 2). An exhaustive protein library vs. target library approach was undertaken to engineer locally this part of the DNA binding interface.

In a first library, the I-*Cre*I scaffold was mutated from D75 to N to decrease likely energetic strains caused by the replacement of the basic residues R68 and R70 in the library that satisfy the hydrogen-acceptor potential of the buried D75 in the I-*Cre*I structure. The D75N mutation did not affect the protein structure, but decreased the toxicity of I-*Cre*I in overexpression experiments. Next, positions 44, 68 and 70 were randomized.

In a second library, the I-*Cre*I scaffold was mutated from R70 to S and I24 to V (I-*Cre*I V24, S70); these mutations did not affect the protein structure. Next, positions 44, 68, 75 and 77 were randomized.

64 palindromic targets resulting from substitutions in positions ±3, ±4 and ±5 of a palindromic target cleaved by I-*Cre*I (Chevalier *et al.,* 2003, precited) were generated, as described in figure 13A.

A robot-assisted mating protocol was used to screen a large number of meganucleases from our library. The general screening strategy is described in figure 4b.

The results from the library of I-*Cre*I N75 mutants having variation at positions 44, 68 and 70 are detailed hereafter. 13,824 meganuclease expressing clones (about 2.3-fold the theoretical diversity) were spotted at high density (20 spots/cm²) on nylon filters and individually tested against each one of the 64 target strains (884,608 spots). 2100 clones showing an activity against at least one target were isolated (Figure 4b) and the ORF encoding the meganuclease was amplified by PCR and sequenced. 410 different sequences were identified and a similar number of corresponding clones were chosen for further analysis. The spotting density was reduced to 4 spots/cm² and each clone was tested against the 64 reporter strains in quadruplicate, thereby creating complete profiles (as in figure 5). 350 positives could be confirmed. Next, to avoid the possibility of strains containing more than one clone, mutant ORFs were amplified by PCR, and recloned in the yeast vector. The resulting plasmids were individually transformed back into yeast. 294 such clones were obtained and tested at low density (4 spots/cm²). Differences with primary screening were observed mostly for weak signals, with 28 weak cleavers appearing now as negatives. Only one positive clone displayed a pattern different from what was observed in the primary profiling.

The 350 validated clones showed very diverse patterns. Some of these new profiles shared some similarity with the wild type scaffold whereas many others were totally different. Various examples are shown on figure 5. Homing endonucleases can usually accommodate some degeneracy in their target sequences, and one of the first findings was that the original I-*Cre*I protein itself cleaves seven different targets in yeast. Many of the mutants followed this rule as well, with the number of cleaved sequences ranging from 1 to 21 with an average of 5.0 sequences cleaved (standard deviation = 3.6). Interestingly, in 50 mutants (14 %), specificity was altered so that they cleaved exactly one target. 37 (11 %) cleaved 2 targets, 61 (17 %) cleaved 3 targets and 58 (17 %) cleaved 4 targets. For 5 targets and above, percentages were lower than 10 %. Altogether, 38 targets were cleaved by the mutants (Figure 6a). It is noteworthy that cleavage was barely observed on targets with an A in position ±3, and never with targets with TGN (tgn) and CGN (cgn) at position ±5, ±4, ±3.

### Example 2: Hierarchical clustering of the variants at positions 44, 68 and/or 70 defines seven I-CreI variant families.

### A) Material and methods

Clustering was done using helust from the R package, and the quantitative data from the primary, low density screening. Both variants and targets were clustered using standard hierarchical clustering with Euclidean distance and Ward's method (Ward, J.H., American Stat. Assoc., 1963, 58, 236-244). Mutants and targets dendrograms were reordered to optimize positions of the clusters and the mutant dendrogram was cut at the height of 8 to define the cluster.

### B) Results

Next, hierarchical clustering was used to determine whether families could be identified among the numerous and diverse cleavage patterns of the variants. Since primary and secondary screening gave congruent results, quantitative data from the first round of yeast low density screening was used for analysis, to permit a larger sample size. Both variants and targets were clustered using standard hierarchical clustering with Euclidean distance and Ward's method (Ward, J.H., precited) and seven clusters were defined (Figure 6c). Detailed analysis is shown for 3 of them (Figure 6b) and the results are summarized in Table I.

**Table I: Cluster Analysis**

| | | Three preferred targets¹ | | Nucleotide in position 4 | | preferred amino acid² | | |
|---|---|---|---|---|---|---|---|---|
| cluster | examples | | | | | | | |
| | (Fig. 3a) | sequence | % cleavage | (%)¹ | | 44 | 68 | 70 |
| 1 | QAN | GTT | 46.2 | G | 0.5 | Q | | |
| | | GTC | 18.3 | A | 2.0 | 80.5% | | |
| 77 proteins | | GTG | 13.6 | T | 82.4 | (62/77) | | |
| | | | Σ= 78.1 | C | 15.1 | | | |
| 2 | QRR | GTT | 13.4 | G | 0 | Q | R | |
| | | GTC | 11.8 | A | 4.9 | 100.0% | 100.0% | |
| 8 proteins | | TCT | 11.4 | T | 56.9 | (8/8) | (8/8) | |
| | | | Σ= 36.6 | C | 38.2 | | | |
| 3 | ARL | GAT | 27.9 | G | 2.4 | A | R | |
| | | TAT | 23.2 | A | 88.9 | 63.0% | 33.8% | |
| 65 proteins | | GAG | 15.7 | T | 5.7 | (41/65) | (22/65) | |
| | | | Σ= 66.8 | C | 3.0 | | | |
| 4 | AGR | GAC | 22.7 | G | 0.3 | A&N | R | R |
| | | TAC | 14.5 | A | 91.9 | 51.6% & 35.4% | 48.4% | 67.7% |
| 31 proteins | | GAT | 13.4 | T | 6.6 | (16&11/31) | 15/31 | 21/31 |
| | | | Σ= 50.6 | C | 1.2 | | | |
| 5 | ADK | GAT | 29.21 | G | 1.6 | | | |
| | DRK | TAT | 15.4 | A | 73.8 | | | |
| 81 proteins | | GAC | 11.4 | T | 13.4 | | | |
| | | | Σ= 56.05.9 | C | 11.2 | | | |
| 6 | KTG | CCT | 30.1 | G | 0 | K | | |
| | RAT | TCT | 19.6 | A | 4.0 | 62.7% | | |
| 51 proteins | | TCC | 13.9 | T | 6.3 | (32/51) | | |
| | | | Σ= 63.6 | C | 89.7 | | | |
| 7 | | CCT | 20.8 | G | 0 | K | | |
| | | TCT | 19.6 | A | 0.2 | 91.9% | | |
| 37 proteins | | TCC | 15.3 | T | 14.4 | (34/37) | | |
| | | | Σ= 55.7 | C | 85.4 | | | |

| | | | | | | | | |
|---|---|---|---|---|---|---|---|---|
| ¹ frequencies according to the cleavage index, as described in Figure 6b ² in each position, residues present in more than 1/3 of the cluster are indicated | | | | | | | | |

For each cluster, a set of preferred targets could be identified on the basis of the frequency and intensity of the signal (Figure 6b). The three preferred targets for each cluster are indicated in Table I, with their cleavage frequencies. The sum of these frequencies is a measurement of the specificity of the cluster. For example, in cluster 1, the three preferred targets (gtt/c/g), account for 78.1% of the observed cleavage, with 46.2% for gtt alone, revealing a very narrow specificity. Actually, this cluster includes several proteins which, as QAN, which cleaves mostly gtt (Figure 5). In contrast, the three preferred targets in cluster 2 represent only 36.6% of all observed signals. In accordance with the relatively broad and diverse patterns observed in this cluster, QRR cleaves 5 targets (Figure 5), while other cluster members' activity are not restricted to these 5 targets.

Analysis of the residues found in each cluster showed strong biases for position 44: Q is overwhelmingly represented in clusters 1 and 2, whereas A and N are more frequent in clusters 3 and 4, and K in clusters 6 and 7. Meanwhile, these biases were correlated with strong base preferences for DNA positions ±4, with a large majority of t:a base pairs in cluster 1 and 2, a:t in clusters 3, 4 and 5, and c:g in clusters 6 and 7 (see Table I). The structure of I-*Cre*I bound to its target shows that residue Q44 interacts with the bottom strand in position -4 (and the top strand of position +4, see Figure 2). These results suggests that this interaction is largely conserved in our mutants, and reveals a "code", wherein Q44 would establish contact with adenine, A44 (or less frequently N44) with thymine, and K44 with guanine. Such correlation was not observed for positions 68 and 70.

### Example 3: Variants can be assembled in functional heterodimers to cleave new DNA target sequences

### A) Materials and Methods

The 75 hybrid targets sequences were cloned as follows: oligonucleotides were designed that contained two different half sites of each mutant palindrome (PROLIGO). Double-stranded target DNA, generated by PCR amplification of the single stranded oligonucleotides, was cloned using the Gateway protocol (INVITROGEN) into yeast and mammalian reporter vectors. Yeast reporter vectors were transformed into *S. cerevisiae* strain FYBL2-7B (*MAT*α*, ura3* Δ*851, trp1* Δ*63, leu2*Δ*1, Iys2*Δ*202*).

### B) Results

Variants are homodimers capable of cleaving palindromic sites. To test whether the list of cleavable targets could be extended by creating heterodimers that would cleave hybrid cleavage sites (as described in figure 7), a subset of I-*Cre*I variants with distinct profiles was chosen and cloned in two different yeast vectors marked by *LEU2* or *KAN* genes. Combinations of mutants having mutations at positions 44, 68 and/or 70 and N at position 75, were then co-expressed in yeast with a set of palindromic and non-palindromic chimeric DNA targets. An example is shown on figure 8: co-expression of the K44, T68, G70, N75 (KTG) and Q44, A68, N70, N75 (QAN) mutants resulted in the cleavage of two chimeric targets, gtt/gcc and gtt/cct, that were not cleaved by either mutant alone. The palindromic gtt, cct and gcc targets (and other targets of KTG and QAN) were also cleaved, likely resulting from homodimeric species formation, but unrelated targets were not. In addition, a gtt, cct or gcc half-site was not sufficient to allow cleavage, since such targets were fully resistant (see ggg/gcc, gat/gcc, gcc/tac, and many others, on figure 8). Unexpected cleavage was observed only with gtc/cct and gtt/gtc, with KTG and QAN homodimers, respectively, but signal remained very weak. Thus, efficient cleavage requires the cooperative binding of two mutant monomers. These results demonstrate a good level of specificity for heterodimeric species.

Altogether, a total of 112 combinations of 14 different proteins were tested in yeast, and 37.5% of the combinations (42/112) revealed a positive signal on their predicted chimeric target. Quantitative data are shown for six examples on figure 9a, and for the same six combinations, results were confirmed in CHO cells in transient co-transfection experiments, with a subset of relevant targets (figure 9b). As a general rule, functional heterodimers were always obtained when one of the two expressed proteins gave a strong signal as homodimer. For example, DRN and RRN, two low activity mutants, give functional heterodimers with strong cutters such as KTG or QRR (Figure 9a and 9b) whereas no cleavage of chimeric targets could be detected by co-expression of the same weak mutants.

### Example 4: Functional endonucleases with new specificity towards nucleotides ±8 to ±10 (10NNN)

The variants are generated according to the experimental procedures described in example 1.

### A) Material and methods

### a) Construction of mutant libraries

I-*Cre*I wt (I-*Cre*I D75), I-*Cre*I D75N (I-*Cre*I N75) and I-*CreI* S70 N75 open reading frames were synthesized, as described previously (Epinat et al., N.A.R., 2003, 31, 2952-2962). Combinatorial libraries were derived from the I-*Cre*I N75, I-*Cre*I D75 and I-*Cre*I S70 N75 scaffolds, by replacing different combinations of residues, potentially involved in the interactions with the bases in positions ± 8 to 10 of one DNA target half-site (Q26, K28, N30, S32, Y33, Q38 and S40). The diversity of the meganuclease libraries was generated by PCR using degenerated primers harboring a unique degenerated codon at each of the selected positions.

Mutation D75N was introduced by replacing codon 75 with aac. Then, the three codons at positions N30, Y33 and Q38 (Ulib4 library) or K28, N30 and Q38 (Ulib5 library) were replaced by a degenerated codon VVK (18 codons) coding for 12 different amino acids: A,D,E,G,H,K,N,P,Q,R,S,T). In consequence, the maximal (theoretical) diversity of these protein libraries was 12³ or 1728. However, in terms of nucleic acids, the diversity was 18³ or 5832.

In Lib4, ordered from BIOMETHODES, an arginine in position 70 of the I-*Cre*I N75 scaffold was first replaced with a serine (R70S). Then positions 28, 33, 38 and 40 were randomized. The regular amino acids (K28, Y33, Q38 and S40) were replaced with one out of 10 amino acids (A,D,E,K,N,Q,R,S,T,Y). The resulting library has a theoretical complexity of 10000 in terms of proteins.

In addition, small libraries of complexity 225 (15²) resulting from the randomization of only two positions were constructed in an I-*Cre*I N75 or I-*Cre*I D75 scaffold, using NVK degenerate codon (24 codons, amino acids ACDEGHKNPQRSTWY).

Fragments carrying combinations of the desired mutations were obtained by PCR, using a pair of degenerated primers coding for 10, 12 or 15 different amino acids, and as DNA template, the I-*Cre*I N75 (Figure 10A), I-*Cre*I D75 or I-*Cre*I S70 N75 open reading frames (ORF). For example, figure 10B illustrates the two pair of primers (Ulib456for and Ulib4rev; Ulib456for and Ulib5rev) used to generate the Ulib4 and Ulib5 libraries, respectively. The corresponding PCR products were cloned back into the I-*Cre*I N75, I-*Cre*I D75 or I-*Cre*I S70 N75 ORF, in the yeast replicative expression vector pCLS0542 (Epinat *et al.,* precited), carrying a *LEU2* auxotrophic marker gene. In this 2 micron-based replicative vector, I-*Cre*I variants are under the control of a galactose inducible promoter.

### b) Construction of target clones

The 64 palindromic targets derived from C1221 were constructed as described in example 1, by using 64 pairs of oligonucleotides (ggcatacaagtttcnnnacgtcgtacgacgtnnngacaatcgtctgtca (SEQ ID NO: 28) and reverse complementary sequences).

### c) Sequence

The open reading frame (ORF) of positive clones identified during the first and/or secondary screening in yeast was amplified by PCR on yeast colonies using primers: PCR-Ga110-F (gcaactttagtgctgacacatacagg, SEQ ID NO: 29) and PCR-Gal10-R (acaaccttgattgcagacttgacc, SEQ ID NO: 30).

### d) Structure analyses

All analyses of protein structures were realized using Pymol. The structures from I-*Cre*I correspond to pdb entry 1g9y. Residue numbering in the text always refer to these structures, except for residues in the second I-*Cre*I protein domain of the homodimer where residue numbers were set as for the first domain.

### B) Results

I-*Cre*I is a dimeric homing endonuclease that cleaves a 22 bp pseudo-palindromic target. Analysis of I-*Cre*I structure bound to its natural target has shown that in each monomer, eight residues establish direct interactions with seven bases (Jurica *et al.,* 1998, precited). According to these structural data, the bases of the nucleotides in positions ± 8 to 10 establish direct contacts with I-*Cre*I amino-acids N30, Y33, Q38 and indirect contacts with I-*Cre*I amino-acids K28 and S40 (Figure 2). Thus, novel proteins with mutations in positions 30, 33 and 38 could display novel cleavage profiles with the 64 targets resulting from substitutions in positions ± 8, ± 9 and ± 10 of a palindromic target cleaved by I-*Cre*I (10NNN target). In addition, mutations might alter the number and positions of the residues involved in direct contact with the DNA bases. More specifically, positions other than 30, 33, 38, but located in the close vicinity on the folded protein, could be involved in the interaction with the same base pairs.

An exhaustive protein library vs. target library approach was undertaken to engineer locally this part of the DNA binding interface. Randomization of 5 amino acids positions would lead to a theoretical diversity of 20⁵ = 3.2x10⁶. However, libraries with lower diversity were generated by randomizing 2, 3 or 4 residues at a time, resulting in a diversity of 225 (15²), 1728 (12³) or 10,000 (10⁴). This strategy allowed an extensive screening of each of these libraries against the 64 palindromic 10NNN DNA targets using a yeast based assay described previously (Epinat *et al.,* 2003, precited and International PCT Application WO 2004/067736) and whose principle is described in Figure 4.

First, the I-*Cre*I scaffold was mutated from D75 to N. The D75N mutation did not affect the protein structure, but decreased the toxicity of I-*Cre*I in overexpression experiments.

Next the Ulib4 library was constructed : residues 30, 33 and 38, were randomized, and the regular amino acids (N30, Y33, and Q38) replaced with one out of 12 amino acids (A,D,E,G,H,K,N,P,Q,R,S,T). The resulting library has a complexity of 1728 in terms of protein (5832 in terms of nucleic acids).

Then, two other libraries were constructed : Ulib5 and Lib4. In Ulib5, residues 28, 30 and 38, were randomized, and the regular amino acids (K28, N30, and Q38) replaced with one out of 12 amino acids (ADEGHKNPQRST). The resulting library has a complexity of 1728 in terms of protein (5832 in terms of nucleic acids). In Lib4, an Arginine in position 70 was first replaced with a Serine. Then, positions 28, 33, 38 and 40 were randomized, and the regular amino acids (K28, Y33, Q38 and S40) replaced with one out of 10 amino acids (A,D,E,K,N,Q,R,S,T,Y). The resulting library has a complexity of 10000 in terms of proteins.

In a primary screening experiment, 20000 clones from Ulib4, 10000 clones from Ulib5 and 20000 clones from Lib4 were mated with each one of the 64 tester strains, and diploids were tested for beta-galactosidase activity. All clones displaying cleavage activity with at least one out of the 64 targets were tested in a second round of screening against the 64 targets, in quadriplate, and each cleavage profile was established, as shown on Figure 11. Then, meganuclease ORFs were amplified from each strain by PCR, and sequenced.

After secondary screening and sequencing of positives over the entire coding region, a total of 1484 unique mutants were isolated showing a cleavage activity against at least one target. Different patterns could be observed. Figure 12 illustrates 37 novel targets cleaved by a collection of 141 variants, including 34 targets which are not cleaved by I-*Cre*I and 3 targets which are cleaved by I-*Cre*I (aag, aat and aac). Twelve examples of profile, including I-*Cre*I N75 and I-*Cre*I D75 are shown on Figure 11A. Some of these new profiles shared some similarity with the wild type scaffold whereas many others were totally different. Homing endonucleases can usually accommodate some degeneracy in their target sequences, and the I-*Cre*I and I-*Cre*I N75 proteins themselves cleave a series of sixteen and three targets, respectively. Cleavage degeneracy was found for many of the novel endonucleases, with an average of 9.9 cleaved targets per mutant (standard deviation: 11). However, among the 1484 mutants identified, 219 (15 %) were found to cleave only one DNA target, 179 (12 %) cleave two, and 169 (11 %) and 120 (8 %) were able to cleave 3 and 4 targets respectively. Thus, irrespective of their preferred target, a significant number of I-*Cre*I derivatives display a specificity level that is similar if not higher than that of the I-*Cre*I N75 mutant (three 10NNN target sequences cleaved), or I-*Cre*I (sixteen 10NNN target sequences cleaved). Also, the majority of the mutants isolated for altered specificity for 10NNN sequences no longer cleave the original C1221 target sequence described in Figure 2 (61 % and 59 %, respectively).

Altogether, this large collection of mutants allowed the targeting of all of the 64 possible DNA sequences differing at positions ±10, ±9, and ±8 (Figure 11B). However, there were huge variations in the numbers of mutants cleaving each target (Figure 11B), these numbers ranged from 3 to 936, with an average of 228.5 (standard deviation: 201.5). Cleavage was frequently observed for targets with a guanine in ±8 or an adenine in ±9, whereas a cytosine in ±10 or ±8 was correlated with low numbers of cleavers. In addition, all targets were not cleaved with the same efficiency. Since significant variations of signal could be observed for a same target, depending on the mutant (compare cleavage efficiencies for the wild type 10AAA target in Figure 11B, for example), an average cleavage efficiency was measured for each target as previously reported (Arnould et al., J. Mol. Biol., 2006, 355, 443-458). These average efficiencies are represented by grey levels on Figure 11B. Analysis of the results show a clear correlation between this average efficiency and the numbers of cleavers, with the most frequently cut target being also the most efficiently cut (compare for example 10TCN, 10CTN and 10CCN targets with 10GAN, 10AAN and 10TAN in Figure 11B).

Thus, hundreds of novel variants were obtained, including mutants with novel substrate specificity ; these variants can keep high levels of activity and the specificity of the novel proteins can be even narrower than that of the wild-type protein for its target.

### Example 5: Statistical analysis of interactions between I-CreI variants at positions 28, 30, 33, 38 and 40 and their targets

### A) Material and methods

Hierarchical clustering was used to establish potential correlations between specific protein residues and target bases, as previously described (Arnould et al., J. Mol. Biol., 2006, 355, 443-458). Clustering was done on the quantitative data from the secondary screening, using helust from the R package. Variants were clustered using standard hierarchical clustering with Euclidean distance and Ward's method (Ward, J.H., American Statist. Assoc., 1963, 58, 236-244). Mutant dendrogram was cut at the height of 17 to define the clusters. For the analysis, cumulated intensities of cleavage of a target within a cluster was calculated as the sum of the cleavage intensities of all cluster's mutants with this target, normalized to the sum of the cleavage intensities of all cluster's mutants with all targets.

### B) Results

Ten different mutant clusters were identified (Table II).

**Table II: Cluster analysis**

| cluster | preferred targets | | nucleotide | | nucleotide | | nucleotide | | preferred amino acids (%) | | | | |
|---|---|---|---|---|---|---|---|---|---|---|---|---|---|
| (effectif) | 10NNN | (%) | -10 (%) | | -9 (%) | | -8 (%) | | 28 | 30 | 33 | 38 | 40 |
| | | | | | | | | | K | | | | |
| 1 | GGG | 7,1 | A | 27,0 | A | 45,9 | A | 21,3 | 100 | N 45.5 | H | Q | S |
| (44) | GAG | 6,9 | C | 4,7 | C | 14,5 | C | 14,7 | 100 | 45,5 | 38,6 | 70,5 | 86,4 |
| | GAT | 6,4 | G | 63,0 | G | 27,9 | G | 37,0 | | K | R | | |
| | | S=20.4 | T | 5,3 | T | 11,8 | T | 27,0 | | 15,9 | 25,0 | | |
| | | | | | | | | | | R | | | |
| | | | | | | | | | | 15,9 | | | |
| 2 | AAG | 6,1 | A | 33,4 | A | 52,2 | A | 20,4 | K | N | G | Q | S |
| (82) | TAG | 5,6 | C | 11,7 | C | 9,4 | C | 13,8 | 100 | 64,6 | 23,2 | 68,3 | 79,3 |
| | GAG | 5,2 | G | 23,7 | G | 19,9 | G | 41,7 | | | | | |
| | | S=16.9 | T | 31,2 | T | 18,5 | T | 24,0 | | | | | |
| 3 | TAG | 4,5 | A | 24,7 | A | 45,2 | A | 19,5 | K | N | T | Q | S |
| (36) | TAC | 4,4 | C | 13,9 | C | 6,7 | C | 16,2 | 100 | 75,0 | 61,1 | 86,1 | 75,0 |
| | TGG | 4,3 | G | 15,8 | G | 26,9 | G | 37,6 | | | C | | |
| | | S=13.2 | T | 45,6 | T | 21,3 | T | 26,7 | | | 22,2 | | |
| 4 | GGG | 30,6 | A | 33,1 | A | 22,6 | A | 10,2 | K | N | R | R | S |
| (74) | AGG | 15,0 | C | 2,6 | C | 1,3 | C | 1,5 | 93,2 | 82,4 | 17,6 | 26,0 | 83,6 |
| | AAG | 7,6 | G | 56,3 | G | 66,2 | G | 77,7 | | | Y | K | |
| | | S=53.2 | T | 8,1 | T | 9,9 | T | 10,6 | | | 16,2 | 19,2 | |
| 5 | GAG | 12,0 | A | 30,0 | A | 71,6 | A | 20,6 | K | N | R | Q | S |
| (115) | GAT | 11,8 | C | 5,1 | C | 5,1 | C | 15,5 | 98,3 | 64,4 | 23,5 | 94,7 | 66,1 |
| | GAA | 8,8 | G | 58,5 | G | 18,4 | G | 35,6 | | | H | | |
| | | S=32.6 | T | 6,4 | T | 5,0 | T | 28,2 | | | 20,9 | | |
| | | | | | | | | | | | Y | | |
| | | | | | | | | | | | 19,1 | | |
| 6 | AAG | 9,1 | A | 40,6 | A | 59,9 | A | 15,7 | K | N | P | Q | S |
| (110) | TAG | 8,7 | C | 9,6 | C | 11,0 | C | 12,2 | 100 | 87,3 | 22 | 68,8 | 61,5 |
| | GAG | 8,0 | G | 23,6 | G | 15,7 | G | 49,0 | | | | | |
| | | S=25.8 | T | 26,2 | T | 13,4 | T | 23,2 | | | | | |
| 7 | AAT | 23,7 | A | 74,9 | A | 85,2 | A | 25,0 | K | N | Y | Q | S |
| (106) | AAA | 16,8 | C | 17,2 | C | 2,5 | C | 11,3 | 86,8 | 41,5 | 92,5 | 95,2 | 70,5 |
| | AAG | 16,6 | G | 5,9 | G | 11,5 | G | 30,0 | | T | | | |
| | | S=57.1 | T | 2,0 | T | 0,8 | T | 33,7 | | 24,5 | | | |
| 8 | GGG | 14,0 | A | 35,5 | A | 41,2 | A | 12,6 | K | N | Y | Q | S |
| (384) | TAG | 10,2 | C | 12,4 | C | 9,1 | C | 12,2 | 89,8 | 63,3 | 45,8 | 43,4 | 62,9 |
| | AAT | 6,9 | G | 30,7 | G | 37,3 | G | 52,4 | | | | | |
| | | S=31.1 | T | 21,4 | T | 12,4 | T | 22,8 | | | | | |
| 9 | TAG | 17,6 | A | 21,1 | A | 62,3 | A | 12,0 | K | N | C | Q | S |
| (134) | TAT | 9,9 | C | 13,0 | C | 2,2 | C | 13,9 | 92,5 | 76,1 | 17,9 | 74,6 | 72,7 |
| | AAG | 7,5 | G | 9,6 | G | 22,1 | G | 51,3 | | | S | | |
| | | S=35.0 | T | 56,3 | T | 13,4 | T | 22,7 | | | 16,4 | | |
| 10 | AAG | 20,2 | A | 64,3 | A | 78,9 | A | 18,1 | K | N | Y | Q | S |
| (399) | AAT | 14,7 | C | 5,4 | C | 6,7 | C | 10,6 | 96,0 | 59,5 | 53,6 | 69,2 | 70,1 |
| | AAA | 10,7 | G | 25,2 | G | 10,4 | G | 44,8 | | | | | |
| | | S=45.6 | T | 5,1 | T | 4,0 | T | 26,5 | | | | | |

| | | | | | | | | | | | | | |
|---|---|---|---|---|---|---|---|---|---|---|---|---|---|
| ¹Target and base frequencies correspond to cumulated intensity of cleavage as described in Materials and Methods). ² In each position, residues present in more than 15% of the cluster are indicated | | | | | | | | | | | | | |

Analysis of the residues found in each cluster showed strong biases for all randomized positions. None of the residues is mutated in all libraries used in this study, and the residues found in the I-*Cre*I scaffold were expected to be overrepresented. Indeed, K28, N30 and S40 were the most frequent residues in all 10 clusters, and no conclusion for DNA/protein interactions can really be infered. However, Y33 was the most represented residue only in clusters 7, 8 and 10, whereas strong occurrence of other residues, such as H, R, G, T, C, P or S, was observed in the seven other clusters. The wild type Q38 residue was overrepresented in all clusters but one, R and K being more frequent in cluster 4.

Meanwhile, strong correlations were observed between the nature of residues 33 and 38 and substrate discrimination at positions ±10 and ±9 of the target.

Prevalence of Y33 was associated with high frequencies of adenine (74.9% and 64.3% in clusters 7 and 10, respectively), and this correlation was also observed, although to a lesser extent in clusters 4, 5 and 8. H33 or R33 were correlated with a guanine (63.0 %, 56.3 % and 58.5 %, in clusters 1, 4 and 5, respectively) and T33, C33 or S33 with a thymine (45.6 % and 56.3 % in clusters 3 and 9, respectively). G33 was relatively frequent in cluster 2, the cluster with the most even base representation in ±10. These results are consistent with the observations of Seligman and collaborators (Nucleic Acids Res., 2002, 30, 3870-3879), who showed previously that a Y33R or Y33H mutation shifted the specificity of I-*Cre*I toward a guanine and Y33C, Y33T, Y33S (and also Y33L) towards a thymine in position ±10.

In addition, R38 and K38 were associated with an exceptional high frequency of guanine in cluster 4, while in all the other clusters, the wild type Q38 residue was overrepresented, as well as an adenine in ±9 of the target.

The structure of I-*Cre*I bound to its target (Chevalier et *al.,* 2003, precited; Jurica et al., 1998, precited) has shown that Y33 and Q38 contact two adenines in -10 and -9 (Figure 2), and the results suggest that these interactions are probably maintained in many of the mutants. Similar results have been described previously for residue 44 and position ±4 (Arnould *et al*., precited). However, comparing the results obtained for the 33/±10, 38/±9 and 44/±4 couples, shows that a given base can be correlated with different amino acid residues, depending on the position. For a guanine, the residues found mostly are R and H in position 33, R or K in 38, and K in 44, for adenine, Y in 33 and Q in 38 and 44, and for thymine, S, C or T in 33 and A in 44. In the three cases, no clear pattern is observed for cytosine. Thus, there is no universal "code", but rather a series of solutions for contacting each base, the best solution depending on a more general context, very similar to what has been observed with Zinc Finger proteins (Pabo *et al.,* precited).

### Example 6: Two I-CreI functional subdomains can behave independantly in terms of DNA binding.

This example shows that an I-*Cre*I target can be separated in two parts, bound by different subdomains, behaving independently. In the I-*Cre*I DNA target, positions ±5, ±4 and ±3 are bound by residues 44, 68 and 70 (Figure 2). Several I-*Cre*I variants, mutated in positions 44, 68, 70 and 75, obtained as described in example 1, were shown to display a detectable activity on C1221, a palindromic target cleaved by I-*Cre*I wild-type (Chevalier, *et al.,* 2003), but were cleaving other targets with various efficacies. In the external part of the binding site, positions ±9 and ±8 are contacted by residues 30, 33 and 38 (Figure 2). A shown on figure 13, the two set of residues are in distinct parts of the proteins. There is no direct interaction with bases ±8. If positions ±5 to ±3 and ±10 to ±8 are bound by two different, independent functional subdomains, engineering of one subdomain should not impact the binding properties of the other domain.

In order to determine if positions ±5 to ±3 and ±9 to ±8 are bound by two different, independent functional subdomains, mutants with altered specificity in the ±5 to ±3 region, but still binding C1221, were assayed for their cleavage properties in the ±10 to ±8 region.

### A) Material and Methods

### a) Structure analyses

All analyses of protein structures were realized using Pymol. The structures from I-*Cre*I correspond to pdb entry 1g9y. Residue numbering in the text always refer to these structures, except for residues in the second I-*Cre*I protein domain of the homodimer where residue numbers were set as for the first domain.

### b) I-CreI variant expressing yeast strain

Mutants were generated as described in examples 1, by mutating positions 44, 68, 70 and 75, and screening for clones able to cleave C1221 derived targets. Mutant expressing plasmids are transformed into S. *cerevisiae* strain FYC2-6A (*MAT*α, *trp1*Δ*63, leu2*Δ1, *his3*Δ*200*).

### c) Construction of target clone

The 64 palindromic targets derived from C1221 by mutation in ±5 to ±3 were constructed as described in example 1, by using 64 pairs of oligonucleotides (ggcatacaagtttcaaaacnnngtacnnngttttgacaatcgtctgtca (SEQ ID NO:31) and reverse complementary sequences).

### d) Mating of meganuclease expressing clones and screening in yeast

Mating was performed as described in example 1, using a low gridding density (about 4 spots/cm²).

### B) Results

64 targets corresponding to all possible palindromic targets derived from C1221 were constructed by mutagenesis of bases ±10 to ±8, as shown on figure 14B. The I-*Cre*I N75 cleavage profile was established, showing a strong signal with the aaa and aat targets, and a weaker one with the aag target.

As shown on figure 14C, proteins with a clearly different cleavage profile in ±5 to ±3, such as QAR, QNR, TRR, NRR, ERR and DRR have a similar profile in ±10 to ±8. The aaa sequence in ±10 to ±8 corresponds to the C1221 target, and is necessarily cleaved by all our variants cleaving C1221. aat is cleaved as well in most mutants (90 %), whereas aag is often not observed, probably because the signal drops below the detection level in faint cleaver. No other target is ever cleaved. These results show that the ±5 to ±3 and ±10 to ±8 regions are bound by two different, largely independent binding units.

### Example 7: Two set of mutations can be combined intramolecularly to obtain novel predictable target specificities as illustrated for the combined target COMB2

### A) General strategy for generating combinatorial mutants capable of cleaving a combined target simultaneously altered at positions 10NNN and 5NNN

The objective here is to determine whether it is possible to combine separable functional subdomains in the I-*Cre*I DNA-binding interface, in order to cleave novel DNA targets.

The identification of distinct groups of mutations in the I-*Cre*I coding sequence that alter the cleavage specificity towards two different regions of the C 1221 target sequence (10NNN (positions - 10 to -8 and + 8 to +10: ± 8 to 10 or ± 10 to 8; example 4) and 5NNN (positions- 5 to -3 and + 3 to +5: ± 3 to 5 or ± 5 to 3; example 1) raises the possibility of combining these two groups of mutants intramolecularly to generate a Combinatorial mutant capable of cleaving a target sequence simultaneously altered at positions 10NNN and 5NNN (Figure 3a).

Positions 28, 30, 33, 38 and 40 on one hand, and 44, 68 and 70, on another hand are on a same DNA-binding fold, and there is no structural evidence that they should behave independently. However, the two sets of mutations are clearly on two spatially distinct regions of this fold (figures 13 and 15) located around different regions of the DNA target. In addition, the cumulative impact of a series of mutations could eventually disrupt the folding. To check whether they are part of two independent functional subunits, mutations from these two series of mutants were combined , and the ability of the resulting variants to cleave the combined target sequence was assayed (Figure 3b).

Therefore, a model non-palindromic target sequence that would be a patchwork of four cleaved 5NNN and 10NNN targets, was designed. This target, COMB1, differs from the C1221 consensus sequence at positions ±3, ±4, ±5, ±8, ±9 and ±10 (Figure 3b). In addition, two derived target sequences representing the left (COMB2) and right (COMB3) halves in palindromic form, were designed (Figure 3b). To generate appropriate I-*Cre*I combinatorial mutants capable of targeting the palindromic targets, mutants efficiently cleaving the 10NNN and 5NNN part of each palindromic sequence were selected (Tables III (this example) and Table IV (example 8), and their characteristic mutations incorporated into the same coding sequence by *in vivo* cloning in yeast (Figure 3b)

Throughout the text and figures, combinatorial mutants for COMB sequences are named with an eight letter code, after residues at positions 28, 30, 33, 38, 40, 44, 68 and 70 (For example, NNSRK/AAR stands for I-*Cre*I 28N30N33S38R40K44A68A70R75N). Parental controls are named with a five letter or three letter code, after residues at positions 28, 30, 33, 38 and 40 (NNSRK stands for I-*Cre*I 28N30N33S38R40K70S75N) or 44, 68 and 70 (AAR stands for I-*Cre*I 44AQ68A70R75N).

All target sequences described in these examples are 22 or 24 bp palindromic sequences. Therefore, they will be described only by the first 11 or 12 nucleotides, followed by the suffix _P, solely to indicate that (for example, target 5' tcaaaacgtcgtacgacgttttga 3' (SEQ ID NO:1) cleaved by the I-*Cre*I protein, will be called tcaaaacgtcgt_P).

Basically, four series of mutations in the I-*Cre*I monomer were obtained as described in examples 1 and 4, respectively. In a first step, a D75N mutation was introduced in the I-*Cre*I scaffold, in order to decrease likely energetic strains caused by the replacement of the basic residues R68 and R70 in the library that satisfy the hydrogen-acceptor potential of the buried D75 in the I-*Cre*I structure.

In this example, mutants able to cleave the 10NNN part (tctggacgtcgt_P target (SEQ ID NO: 37)) of COMB2 were obtained by mutagenesis of positions 28, 30, 33 or 28, 33, 38, and 40 (Table III), and mutants able to cleave the 5NNN part (tcaaaacgacgt_P (SEQ ID NO:38) of COMB2 were obtained by mutagenesis of positions 44, 68 and 70 cleave (Table III).

In example 8, mutants able to cleave the 10NNN part (tcgatacgtcgt_P (SEQ ID NO:44) of COMB3 were obtained by mutagenesis of positions 28, 30, 33 or 28, 33, 38, and 40 (Table IV), and mutants able to cleave the 5NNN part (tcaaaaccctgt_P (SEQ ID NO:45)) of COMB3 were obtained by mutagenesis of positions 44, 68 and 70 cleave (Table IV).

Then, for each combined target (COMB2 or COMB3), mutations at positions 28, 30, 33, 38 and 40 from mutants cleaving 10NNN targets were combined with mutations at position 44, 68 and 70 from mutants cleaving 5NNN targets, and the ability of the resulting combinatorial mutants to cleave the appropriate target sequence COMB2 (tctggacgacgt_P (SEQ ID NO:39); this example) or COMB3 (tcaaaac**cct**gt_P (SEQ ID NO:45); example 8), was assayed.

### B) Material and Methods:

### a) Construction of combinatorial mutants

In order to generate an I-*Cre*I coding sequence containing mutations derived from different libraries (amino acids 28,30,33,38,40 and 44,68,70 or 44,68,70,75,77), separate overlapping PCR reactions were carried out that amplify the 5' end (aa positions 1-43) or the 3' end (positions 39-167) of the I-*Cre*I coding sequence (Figure 10). For both the 5' and 3' end, PCR amplification is carried out using a primer specific to the vector (pCLS0542) (Gal10F 5'-gcaactttagtgctgacacatacagg-3' (SEQ ID NO:40) or Gal10R 5'-acaaccttgattggagacttgacc-3'(SEQ ID NO:41)) and a primer specific to the I-*Cre*I coding sequence for amino acids 39-43 (assF 5'-ctaxxxttgaccttt-3' (SEQ ID NO:42) or assR 5'-aaaggtcaaxxxtag-3' (SEQ ID NO:43)) where xxx codes for residue 40. The resulting PCR products contain 15 bp of homology with each other and approximately 100-200 bp of homology with the 2 micron-based replicative vectors, pCLS0542, marked with the *LEU2* gene and pCLS1107, containing a kanamycin resistant gene. Thus, to generate an intact coding sequence containing both groups of mutations, by *in vivo* homologous recombination, approximately 25ng of each of the two overlapping PCR fragments and either 25ng of the pCLS0542 vector DNA linearized by digestion with *Nco*I and *Eag*I or 25ng of the pCLS1107 vector DNA linearized by digestion with *Dra*III and *NgoM*IV are used to transform the yeast *Saccharomyces cerevisiae* strain FYC2-6A (*MATα, trp1*Δ*63, leu2*Δ1*, his3*Δ*200*) using a high efficiency LiAc transformation protocol (Gietz and Woods, Methods Enzymol., 2002, 350, 87-96). Combinatorial mutants were generated individually. PCR reactions were pooled in equimolar amounts and transformed into yeast together with the linearized plasmid. Transformants were selected on either synthetic medium lacking leucine (pCLS0542) or rich medium containing G418 (pCLS 1107).

### b) Construction of target clones

Targets were cloned as described in example 1.

### c) Mating of homing endonuclease expressing clones and screening in yeast

Mating of homing endonuclease expressing clones and screening in yeast was performed as described in example 1, using a high gridding density (about 20 spots/cm2).

### C) Results

I-*Cre*I mutants cleaving tc**tgg**acgtcgt_P (SEQ ID NO:37) and tcaaaacgacgt_P (SEQ ID NO: 38) were identified as described in examples 1 and 4. Three variants, mutated in positions 30, 33, 38, 40 and 70, capable of cleaving the sequence tc**tgg**acgtcgt_P (SEQ ID NO:37; Table III) were combined with 31 different variants, mutated in positions 44, 68 and 70, capable of cleaving the sequence tcaaaacgacgt_P (SEQ ID NO:38; Table III). Both set of proteins are mutated in position 70. However, the hypothesis of two separable functional subdomains implies that this position has little impact on the specificity in ±10 to ±8. Therefore, in the combined protein, only the residues 30, 33, 38 and 40 from the first set of proteins were used, residue70 being picked from the second set of proteins.

The resulting 93 mutants were assayed for cleavage in yeast containing a LacZ assay with the combined target sequence COMB2 (tct**gg**acg**a**cgt_P: SEQ ID NO:39). Thirty two combined mutants were capable of cleaving the target (Tableau III and figure 16). Cleavage of the combined target sequence is specific to the combinatorial mutant as each of the parent mutants was unable to cleave the combined sequence (figure 16). In addition, while the parental mutants displayed efficient cleavage of the 5NNN and 10NNN target sequences, all combinatorial mutants but one displayed no significant activity for these sequences (Figure 16), or for the original C1221 sequence. The only exception was NNSRR/ARS, which was found to faintly cleave the 5GAC target (Figure 16).

These results indicate that combining mutations at positions 28, 30, 33, 38, 40 and 44, 68, 70 can give rise to functional endonucleases with the expected specificity for approximately 30% of the tested combinations. This study identifies residues 28-40 on one hand, and 44-70 on another hand, as part of two separable DNA-binding subdomains (Figure 15).

**Table III: Combinatorial mutants* tested against the COMB2 target**

| **Residues 44, 68 and 70¹** | **Residues 28, 30, 33, 38, 40 ²** | | |
|---|---|---|---|
| | **NNSRK** | **NNSRR** | **QNSRK** |
| **AAR** | + | + | + |
| **AGR** | | | |
| **AHR** | + | + | + |
| **AKR** | + | + | + |
| **ANR** | | | |
| **AQR** | + | + | + |
| **ARA** | | | |
| **ARG** | | | |
| **ARH** | | | |
| **ARL** | | | |
| **ARN** | + | + | + |
| **ARR** | + | + | + |
| **ARS** | + | + | |
| **ART** | | | |
| **ASK** | | | |
| **ASR** | + | + | + |
| **ATR** | + | + | + |
| **NAR** | | | |
| **NHR** | | | |
| **NRA** | | | |
| **NRG** | | | |
| **NRR** | + | + | + |
| **NSR** | | | |
| **NTR** | | | |
| **PDT** | | | |
| **QRG** | | | |
| **QTR** | | | |
| **SRR** | | | |
| **SRS** | | | |
| **THR** | | | |
| **TRR** | + | + | + |

| | | | |
|---|---|---|---|
| * Combinatorial mutants are created by assembling mutations in 28, 30, 33, 38, 40, 44, 68 and 70 in an I-*Cre*I N75 scaffold. Combinatorial mutants cleaving COMB2 are indicated by +. ¹ mutations identified in I-*Cre*IN75 variants cleaving the chosen 5GAC target. ² mutations identified in I*-Cre*I S70N75 variants cleaving the 10TGG chosen target. | | | |

### Example 8: Two set of mutations can be combined intramolecularly to obtain novel predictable target specificities, as illustrated with the combined target COMB3

### A) Material and Methods:

The experimental procedures are described in example 7.

### B) Results

Seven variants mutated in positions 28, 33, 38, 40 and 70, and capable of cleaving the sequence tc**g**a**t**acgtcgt_P (SEQ ID NO:44, Table IV) were combined with 30 different variants mutated in positions 44, 68 and 70, and capable of cleaving the sequence tcaaaac**cct**gt_P (SEQ ID NO:45, Table IV). Mutations in position 70 are found in both set of proteins. However, the hypothesis of two separable functional subdomains implies that this position has little impact on the specificity in ±10 to ±8. Therefore, in the combined protein, only the residues 30, 33, 38 and 40 from the first set of proteins were used, residue 70 being picked from the second set of proteins.

The resulting 210 mutants were assayed for cleavage in yeast containing a LacZ assay with the combined target sequence COMB3 (tcgataccctgt_P (SEQ ID NO:46)). Seventy-seven combined mutants were capable of cleaving the target (Table IV). Cleavage of the combined target sequence is specific to the combinatorial mutant as each of the parent mutants was unable to cleave the combined sequence. In addition, while the parental mutants displayed efficient cleavage of the 5NNN and 10NNN target sequences, all combinatorial mutants displayed no significant activity for these sequences or for the original C1221 sequence.

These results indicate that combining mutations at positions 28, 30, 33, 38, 40 and 44, 68, 70 can give rise to functional endonucleases with the expected specificity for approximately 30 % of the tested combinations. This study identifies residues 28-40 on one hand, and 44-70 on another hand, as part of two separable DNA-binding subdomains (Figure 15).

**Table IV: Combinatorial mutants* tested against the COMB3 target.**

| Residues 44, 68 and 70 ¹ | Residues 28, 30, 33, 38 and 40 | | | | | | |
|---|---|---|---|---|---|---|---|
| | ANRQR² | KNRQA² | QNRQK² | QNRQR² | SNRQR² | TNRQR² | KNHQS³ |
| AAK | | | | | | | |
| AGR | | | | | **+** | | |
| ARD | | | | | | | |
| GQT | | | | | | | **+** |
| HAT | | | | | | | |
| HRE | | | | | | | **+** |
| KAD | **+** | | | **+** | | | **+** |
| KAG | | | | | | **+** | **+** |
| KAN | **+** | | | **+** | | | **+** |
| KAS | **+** | **+** | **+** | **+** | **+** | **+** | **+** |
| KDT | | | | **+** | | **+** | **+** |
| KEG | **+** | | | **+** | | **+** | **+** |
| KES | | **+** | | | | **+** | **+** |
| KGT | **+** | | **+** | **+** | | **+** | **+** |
| KHD | | | | | **+** | | **+** |
| KHN | | | | | | | |
| KHS | | | | | | | **+** |
| KND | | **+** | | | | **+** | **+** |
| KNN | | | | | | | |
| KNT | **+** | **+** | **+** | **+** | | | **+** |
| KQS | | **+** | | | | | **+** |
| KRA | **+** | | **+** | **+** | | | **+** |
| KRD | **+** | **+** | **+** | | **+** | **+** | **+** |
| KRG | **+** | | **+** | **+** | | **+** | **+** |
| KRT | **+** | **+** | **+** | | **+** | **+** | **+** |
| KST | **+** | | **+** | **+** | | **+** | **+** |
| KTD | | | | | | | **+** |
| KTS | **+** | | **+** | | | | **+** |
| RAT | | | | | | | **+** |
| SDK | | | | | | | |

| | | | | | | | |
|---|---|---|---|---|---|---|---|
| *Combinatorial mutants are created by assembling mutations in 28, 30, 33, 38, 40, 44, 68 and 70 in an I-Crel N75 scaffold. Combinatorial mutants cleaving COMB3 are indicated by +. ¹ mutations identified in I-CreI N75 variants cleaving the chosen 5CCT target. ² mutations identified in I-CreI S70 N75 variants cleaving the 10GAT chosen target. ³ mutations identified in an I-CreI N75 variant cleaving the 10GAT chosen target. | | | | | | | |

### Example 9: Two set of mutations can be combined intramolecularly to obtain novel predictable target specificities, as illustrated with the combined target SEQ ID NO: 49

The objective here is to determine whether it is possible to identify and combine separable functional subdomains in the I-*Cre*I DNA-binding interface, in order to cleave novel DNA targets. All target sequences described in this example are 24 bp palindromic sequences. Therefore, they will be described only by the first 12 nucleotides, followed by the suffix _P, solely to indicate that (for example, target 5' tcaaaacgtcgtacgacgttttga 3' (SEQ ID NO:1), cleaved by the I-*Cre*I protein, will be called tcaaaacgtcgt_P).

Two series of mutations in the I-*Cre*I monomer were obtained as described in examples 1 and 4. In a first step, a D75N mutation was introduced in the I-CreI scaffold, in order to decrease likely energetic strains caused by the replacement of the basic residues R68 and R70 in the library that satisfy the hydrogen-acceptor potential of the buried D75 in the I-*Cre*I structure. Then mutants able to cleave the tcaa**c**acgtcgt_P (SEQ ID NO:47 ) target were obtained by mutagenesis of positions 28, 30, 33 or 28, 33, 38, and 40, (Table V), and mutants able to cleave tcaaaac**cct**gt_P (SEQ ID NO: 48) were obtained by mutagenesis of positions 44, 68 and 70 cleave (Table V).

Positions 28, 30, 33, 38 and 40 on one hand, and 44, 68 and 70, on another hand are on a same DNA-binding fold, and there is no structural evidence that they should behave independently. However, the two sets of mutations are clearly on two spatially distinct regions of this fold (Figure 15), located around different regions of the DNA target. To check whether they are part of two independent functional subunits, we have combined mutations from these two series of mutants, check whether they could cleave the tcaa**c**ac**cct**gt_P (SEQ ID NO: 49) chimeric target.

### A) Material and Methods:

The experimental procedures are described in example 7.

### B) Results

Five variants, mutated in positions 28, 30, 33, 38, 40 and 70, and capable of cleaving the sequence tcaa**c**acgtcgt_P (SEQ ID NO: 47, Table V) were combined with 34 different variants mutated in positions 44, 68 and 70, and capable of cleaving the sequence tcaaaac**cct**gt_P (SEQ ID NO:48, Table V). Mutations in position 70 are found in both set of proteins. However, the hypothesis of two separable functional subdomains implies that this position has little impact on the specificity in ±10 to ±8. Therefore, in the combined protein, only the residues 30, 33, 38 and 40 from the first set of proteins were used, residue 70 being picked from the second set of proteins. The resulting 170 mutants were assayed for cleavage in yeast containing a LacZ assay with the combined target sequence tcaa**c**ac**cct**gt_P (SEQ ID NO: 49). Thirty seven combined mutants were capable of cleaving the target (figure 17B) whereas only one (I-*Cre*I K44, R68, D70, N75) of the individual mutants was able to cleave the combined sequence (figure 17A). This study identifies residues 28-40 on one hand, and 44-70 on another hand, as part of two separable DNA-binding subdomains (Figure 15).

**Table V: Variants used in this study°**

| **Nickname** | **Aminoacid in position** | | | | | | | | | **Target sequence (SEQ ID NO:1*, 47**, 48)** |
|---|---|---|---|---|---|---|---|---|---|---|
| | **28** | **30** | **33** | **38** | **40** | **44** | **68** | **70** | **75** | |
| **I-CreI** | K | N | Y | Q | S | Q | R | R | D | tcaaaacgtcgt_P* |
| **I-CreI N75** | | | | | | | | | N | tcaaaacgtcgt_P* |
| **KNRQE** | | | R | | E | | | S | N | Tcaa**c**acgtcgt_P** |
| **KNRQQ** | | | R | | Q | | | S | N | Tcaa**c**acgtcgt_P** |
| **RNRYQ** | R | | R | Y | Q | | | S | N | Tcaa**c**acgtcgt_P** |
| **KGYGS** | | G | | G | | | | | N | Tcaa**c**acgtcgt_P** |
| **RNYQS** | R | | | | | | | S | N | Tcaa**c**acgtcgt_P** |
| **KTG** | | | | | | K | T | G | N | tcaaaac**cct**gt_P |
| **KTH** | | | | | | K | T | H | N | tcaaaac**cct**gt_P |
| **PDT** | | | | | | P | D | T | N | tcaaaac**cct**gt_P |
| **NHN** | | | | | | N | H | N | N | tcaaaac**cct**gt_P |
| **AAK** | | | | | | A | A | K | N | tcaaaac**cct**gt_P |
| **ARD** | | | | | | A | | D | N | tcaaaac**cct**gt_P |
| **HAT** | | | | | | H | A | T | N | tcaaaac**cct**gt_P |
| **KAD** | | | | | | K | A | D | N | tcaaaac**cct**gt_P |
| **KAN** | | | | | | K | A | N | N | tcaaaac**cct**gt_P |
| **KDT** | | | | | | K | D | T | N | tcaaaac**cct**gt_P |
| **KES** | | | | | | K | E | S | N | tcaaaac**cct**gt_P |
| **KHD** | | | | | | K | H | D | N | tcaaaac**cct**gt_P |
| **AGR** | | | | | | A | G | | N | tcaaaac**cct**gt_P |
| **GQT** | | | | | | G | Q | T | N | tcaaaac**cct**gt_P |
| **HRE** | | | | | | H | | E | N | tcaaaac**cct**gt_ P |
| **KAG** | | | | | | K | A | G | N | tcaaaac**cct**gt P |
| **KAS** | | | | | | K | A | S | N | tcaaaac**cct**gt_P |
| **KEG** | | | | | | K | E | G | N | tcaaaac**cct**gt_P |
| **KGT** | | | | | | K | G | T | N | tcaaaac**cct**gt_P |
| **KHN** | | | | | | K | H | N | N | tcaaaac**cct**gt_P |
| **KHS** | | | | | | K | H | S | N | tcaaaac**cct**gt_P |
| **KNN** | | | | | | K | N | N | N | tcaaaac**cct**gt_P |
| **KQS** | | | | | | K | Q | S | N | tcaaaac**cct**gt P |
| **KRD** | | | | | | K | | D | N | tcaaaac**cct**gt P |
| **KRT** | | | | | | K | | T | N | tcaaaac**cct**gt_P |
| **KTD** | | | | | | K | T | D | N | tcaaaac**cct**gt_P |
| **RAT** | | | | | | R | A | T | N | tcaaaac**cct**gt_P |
| **KND** | | | | | | K | N | D | N | tcaaaac**cct**gt_P |
| **KNT** | | | | | | K | N | T | N | tcaaaac**cct**gt_P |
| **KRA** | | | | | | K | | A | N | tcaaaac**cct**gt_P |
| **KRG** | | | | | | K | | G | N | tcaaaac**cct**gt_P |
| **KST** | | | | | | K | S | T | N | tcaaaac**cct**gt_P |
| **KTS** | | | | | | K | S | S | N | tcaaaac**cct**gt_P |
| **SDK** | | | | | | S | D | K | N | tcaaaac**cct**gt_P |

| | | | | | | | | | | |
|---|---|---|---|---|---|---|---|---|---|---|
| °I-*Cre*I and I*-Cre*I N75 are indicated as references. Amino-acid residues are indicated only when different from I*-Cre*I*.* | | | | | | | | | | |

### Example 10: Two set of mutations can be combined intramolecularly to obtain novel predictable target specificities, as illustrated with the combined target SEQ ID NO: 52

The objective here is to determine whether it is possible to identity and combine separable functional subdomains in the I-*Cre*I DNA-binding interface, in order to cleave novel DNA targets. All target sequences described in this example are 24 bp palindromic sequences. Therefore, they will be described only by the first 12 nucleotides, followed by the suffix _P, solely to indicate that (for example, target 5' tcaaaacgtcgtacgacgttttga 3' (SEQ ID NO:1 ), cleaved by the I-*Cre*I protein, will be called tcaaaacgtcgt_P).

Two series of mutations in the I-CreI monomer were obtained as described in examples 1 and 4. In a first step, a D75N mutation was introduced in the I-CreI scaffold, in order to decrease likely energetic strains caused by the replacement of the basic residues R68 and R70 in the library that satisfy the hydrogen-acceptor potential of the buried D75 in the I-*Cre*I structure. Then mutants able to cleave the tcaa**c**acgtcgt_P target (SEQ ID NO:50) were obtained by mutagenesis of positions 28, 30, 33 or 28, 33, 38, and 40, (Table VI), and mutants able to cleave tcaaaac**ttt**gt_P (SEQ ID NO: 51) were obtained by mutagenesis of positions 44, 68 and 70 cleave (Table VI).

Positions 28, 30, 33, 38 and 40 on one hand, and 44, 68 and 70, on another hand are on a same DNA-binding fold, and there is no structural evidence that they should behave independently. However, the two sets of mutations are clearly on two spatially distinct regions of this fold (figure 15), located around different regions of the DNA target. To check whether they are part of two independent functional subunits, we have combined mutations from these two series of mutants, check whether they could cleave the tcaacac**ttt**gt_P chimeric target (SEQ ID NO: 52).

### A) Material and Methods:

The experimental procedures are described in example 7.

### B) Results

Five variants mutated in positions 28, 30, 33, 40 and 70, and capable of cleaving the sequence tcaa**c**acgtcgt_P (SEQ ID NO:50) were combined with 29 different variants mutated in positions 44, 68 and 70, and capable of cleaving the sequence tcaaaac**ttt**gt_P (SEQ ID NO:51). Mutations in position 70 are found in both set of proteins. However, the hypothesis of two separable functional subdomains implies that this position has little impact on the specificity in ±10 to ±8. Therefore, in the combined protein, only the residues 30, 33, 38 and 40 from the first set of proteins were used, residue 70 being picked from the second set of proteins. The resulting 145 mutants were assayed for cleavage in yeast containing a LacZ assay with the combined target sequence tcaacac**ttt**gt_P (SEQ ID NO:52). Twenty three active combined mutants were identified. However, for all of them, one parental mutant was also cleaving the target. Nevertheless, this demonstrates a large degree of liberty between the two sets of mutations. Combined mutants capable of cleaving the target were capable of cleaving the combined sequence as individual mutants (figure 18A and B).

**Table VI: Variants used in this study°**

| **Nickname** | **Aminoacid in position** | | | | | | | | | **Target sequence (SEQ ID NO:1*, 50**, 51)** |
|---|---|---|---|---|---|---|---|---|---|---|
| | **28** | **30** | **33** | **38** | **40** | **44** | **68** | **70** | **75** | |
| **I-CreI** | K | N | Y | Q | S | Q | R | R | D | tcaaaacgtcgt_P* |
| **I-CreI N75** | | | | | | | | | N | tcaaaacgtcgt_P* |
| **KNRQE** | | | R | | E | | | S | N | Tcaa**c**acgtcgt_P** |
| **KNRQQ** | | | R | | Q | | | S | N | Tcaa**c**acgtcgt_P** |
| **RNRYQ** | R | | R | Y | Q | | | S | N | Tcaa**c**acgtcgt_P* |
| **KGYGS** | | G | | G | | | | | N | Tcaa**c**acgtcgt_P** |
| **RNYQS** | R | | | | | | | S | N | Tcaa**c**acgtcgt_P* |
| **AAR** | | | | | | A | A | | N | tcaaaac**ttt**gt_P |
| **ARD** | | | | | | A | | D | N | tcaaaac**ttt**gt_P |
| **ERA** | | | | | | E | | A | N | tcaaaac**ttt**gt_P |
| **KAN** | | | | | | K | A | N | N | tcaaaac**ttt**gt_P |
| **KAS** | | | | | | K | A | S | N | tcaaaac**ttt**gt_P |
| **KGA** | | | | | | K | G | A | N | tcaaaac**ttt**gt_P |
| **KDS** | | | | | | K | D | S | N | tcaaaac**ttt**gt_P |
| **KHS** | | | | | | K | H | S | N | tcaaaac**ttt**gt_P |
| **KNA** | | | | | | K | N | A | N | tcaaaac**ttt**gt_P |
| **KQS** | | | | | | K | Q | S | N | tcaaaac**ttt**gt_P |
| **KRA** | | | | | | K | | A | N | tcaaaac**ttt**gt_P |
| **KRD** | | | | | | K | | D | N | tcaaaac**ttt**gt_P |
| **KRG** | | | | | | K | | G | N | tcaaaac**ttt**gt_P |
| **KTE** | | | | | | K | T | E | N | tcaaaac**ttt**gt_P |
| **KTS** | | | | | | K | T | S | N | tcaaaac**ttt**gt_P |
| **NHN** | | | | | | N | H | N | N | tcaaaac**ttt**gt_P |
| **QAK** | | | | | | | A | K | N | tcaaaac**ttt**gt_P |
| **QNH** | | | | | | | N | H | N | tcaaaac**ttt**gt_P |
| **QRA** | | | | | | | | A | N | tcaaaac**ttt**gt_P |
| **QRD** | | | | | | | | D | N | tcaaaac**ttt**gt_P |
| **QRG** | | | | | | | | G | N | tcaaaac**ttt**gt_P |
| **QRH** | | | | | | | | H | N | tcaaaac**ttt**gt_P |
| **QRN** | | | | | | | | N | N | tcaaaac**ttt**gt_P |
| **QRQ** | | | | | | | | Q | N | tcaaaac**ttt**gt_P |
| **QRS** | | | | | | | | S | N | tcaaaac**ttt**gt_P |
| **QSG** | | | | | | | S | G | N | tcaaaac**ttt**gt_P |
| **QSH** | | | | | | | S | S | N | tcaaaac**ttt**gt_P |
| **QSS** | | | | | | | S | S | N | tcaaaac**ttt**gt_P |
| **SDK** | | | | | | S | D | D | N | tcaaaac**ttt**gt_P |

| | | | | | | | | | | |
|---|---|---|---|---|---|---|---|---|---|---|
| °I*-Cre*I and I-*Cre*I N75 are indicated as references. Amino-acid residues are indicated only when different from I-CreI. | | | | | | | | | | |

### Example 11: Biochemical and biophysical analysis of homodimeric combinatorial mutants

### A) Material and Methods

Novel I-*Cre*I variants were expressed, purified, and analyzed for in vitro cleavage as reported previously (Arnould *et al.*, precited). Circular dichroism (CD) measurements were performed on a Jasco J-810 spectropolarimeter using a 0.2 cm path length quartz cuvette. Equilibrium unfolding was induced increasing temperature at a rate of 1°C/min (using a programmable Peltier thermoelectric). Samples were prepared by dialysis against 25 mM potassium phosphate buffer, pH 7.5, at protein concentrations of 20 µM.

### B) Results

Four combinatorial mutants cleaving COMB2 or COMB3, and their corresponding parent mutants were analyzed *in vitro* in order to compare their relative cleavage efficiencies. As can be observed in Figures 19a-c, cleavage of the combined palindromic target sequences (COMB2 or COMB3) is specific to the combinatorial mutants since the two parent mutants were unable to cleave these sequences. In addition, while the parental mutants displayed efficient cleavage of the 5NNN and 10NNN target sequences, only one out of the four combinatorial mutants (NNSRK/ARR) displayed a faint activity on one of these targets, the others being totally inactive. Thus, results from the yeast assay were confirmed *in vitro.* Importantly, the differences in activity levels between mutants were also consistent with the variations observed in yeast, and this congruency was further confirmed by the *in vitro* study of 4 additional mutants cleaving COMB3. Thus, the variations of signal observed in yeast are not due to differences in expression levels, but really reflect differences in binding/and or cleavage properties.

Finally, analysis of the structure and stability of this group of combinatorial mutants was performed using far-UV CD (Figure 19d), ¹H-NMR and analytical ultracentrifugation. All the mutants are dimers and their secondary and tertiary structures as well as thermal denaturation curves (Figure 19d) are similar to that of the original I*-Cre*I N75 protein, showing that engineering did not result in a significant alteration of the structure, folding or stability of these proteins. Thus, the two sets of residues that were mutated, K28, N30, Y33, Q38 and S40 on one hand, Q44, R68 and R70 on the other, define two relatively independent DNA binding subdomains.

### Example 12: Co-expression of combinatorial mutants results in cleavage of chimeric target sites

### A) Material and Methods:

The experimental procedures are described in example 3.

### B) Results

To determine if combinatorial mutants could function efficiently as heterodimers, a subset of mutants capable of cleaving the palindromic sites COMB2 and COMB3 were co-expressed in yeast and assayed for their ability to cleave the chimeric site COMB1, corresponding to the fusion of the two half sites of the original targets (Figure 20a). As can be observed in figure 20a, co-expression resulted in cleavage of the chimeric sequence COMB1 among all tested heterodimers. This activity appears to be specific to the heterodimers since each one of the mutants expressed alone displayed no detectable activity with the chimeric target site (Figure 20a). In general, co-expression of two mutants displaying strong activity for COMB2 and/or COMB3 will result in a higher level of activity for the chimeric site than a coexpression of two mutants displaying weak activity (For example, compare KNHQS/KEG x NNSRK/ARR with QNRQR/KEG x NNSRK/ASR in figure 20a).

Cleavage of the COMB 1 target was also detected *in vitro* when the KNHQS/KAS and NNSRK/ARR purified proteins were incubated together with the COMB 1 target in our conditions, while incubation of single protein did not give rise to any detectable cleavage activity. However, the cleavage efficiency was extremely low, which might result from slow heterodimer formation *in vitro*. Indeed, Silva *et al*. could show that engineered derivatives from I*-Dmo*I had to be coexpressed in *E. coli* to form active heterodimers (Nucleic Acids Res., 2004, 32, 3156-3168) and is not clear whether I-*Cre*I homodimers can exchange subunits easily. Actually, it cannot be excluded that low levels of cleavage could result from an alternative pathway, such as subsequent nicking by the two homodimers in solution, and we are currently investigating this issue.

Altogether, these results indicate that a combinatorial approach can generate artificial HEs capable of effectively cleaving chimeric target sites altered at position 10NNN and 5NNN. The generation of collections of I-CreI derivatives allows today for cleavage of all 64 10NNN targets and 62 out of the 64 5NNN targets (our unpublished data). The ability to combine them intramolecularly as well as intermolecularly, increases the number of attainable 22-mers to at least 1.57 x 10⁷ ((64x62)²)_{.}

### Example 13: Redesigned Homing Endonucleases cleave a natural target sequence in the RAG1 gene.

### A) Material and Methods

The experimental procedures are as described in examples 3 and 7, with the exception that the combinatorial mutants for the RAG target were generated as libraries, in contrast to the combinatorial mutants for the COMB targets (example 7) which were generated individually.

### B) Results

To analyse the effectiveness of a combinatorial approach for designing HEs for natural target sites, the human RAG1 gene was analysed for potential sites compatible with mutants present in the 10NNN and 5NNN libraries. RAG1 has been shown to form a complex with RAG2 that is responsible for the initiation of V(D)J recombination, an essential step in the maturation of immunoglobulins and T lymphocyte receptors (Oettinger et al., Science, 1990, 248, 1517-1523; Schatz et al., Cell, 1989, 59, 1035-1048). Patients with mutations in *RAG1* display severe combined immune deficiency (SCID) due to the absence of T and B lymphocytes. SCID can be treated by allogenic hematopoetic stem cell transfer from a familial donor and recently certain types of SCID have been the subject of gene therapy trials (Fischer et al., Immunol. Rev., 2005, 203, 98-109).

Analysis of the genomic locus of RAG1 revealed a potential target site located 11bp upstream of the coding exon of RAG1, that was called RANG1.1 (Figure 3c). In contrast to the COMB sequence, the RAG1.1 site not only differs from the C1221 site at position 10NNN and 5NNN but also at 11N (11 t instead of 11c) and 7NN (7ct instead of 7ac). I-*Cre*I D75N is tolerant to these changes, and it was speculated that combinatorial mutants would also be tolerant to changes at these positions. For the 5NNN region, the mutants used were from the previously reported library mutated at positions 44, 68, 70 (Arnould *et al.*, precited), as well as from another library mutated at positions 44, 68, 75 and 77, with a serine residue at position 70. Since additional residues were mutated, combinatorial mutants are named after 10 residues instead of 8, the two last letters corresponding to the residues at position 75 and 77 (For example, KNTAK/NYSYN stands for I*-Cre*I 28K30N33T38A40K44N68Y70S75Y77N).

In contrast with the mutants used for COMB targets, which were generated individually, mutants used for RAG targets were generated in libraries. For the RAG1.2 target sequence, a library with a putative complexity of 1300 mutants was generated. Screening of 2256 clones yielded 64 positives (2.8%), which after sequencing, turned out to correspond to 49 unique endonucleases. For RAG1.3, 2280 clones were screened, and 88 positives were identified (3.8%), corresponding to 59 unique endonucleases. In both cases, the combinatorial mutants were unable to cleave the 5NNN and 10NNN target sequences as well as the original C1221. In contrast with COMB mutants, which were generated and tested individually, RAG mutants were generated as libraries. Nevertheless, no obvious bias was detected in these libraries, and these frequencies should be representative of the real frequency of functional positives. This lower success rate, compared with screening with the COMB targets, could be due to the additional mutations at positions 75 and 77, or from the additional changes at positions ±6, ±7 and ±11 in these targets.

As for COMB 1, a panel of mutants able to cleave the palindromic targets was then co-expressed in the yeast to test the RAG1.1 target cleavage. Figure 20b shows that co-expression resulted in the cleavage of the natural target. RAG1.1 target cleavage was due to the heterodimers resulting from co-expression as none of these mutants was able to cleave RAG1.1 when expressed alone (Figure 20b). This is the first time a homing endonuclease is entirely redesigned to cleave a naturally occurring sequence. The making of these combinatorial mutants opens large possibilities for it is the key step towards global engineering of the DNA binding interface of LAGLIDADG proteins.

## Claims

1. An I*-Cre*I homing endonuclease variant having a modified cleavage specificity **characterized in that**:
said variant comprises one or more mutation(s) at positions contacting a DNA target sequence or interacting directly or indirectly with said DNA target; and
said I-*Cre*I variant cleaves said DNA target sequence consisting of a mutant I*-Cre*I site wherein said DNA target comprises a sequence having the formula:
c-₁₁n-₁₀n₋₉n₋₈m₋₇y₋₆n₋₅n₋₄n₋₃k₋₂y₋₁r₊₁m₊₂n₊₃n₊₄n₊₅r₊₆k₊₇n₊₈n₊₉n₊₁₀g₊₁₁ (I), wherein n is a, t, c, or g, m is a or c, y is c or t, k is g or t, and r is a or g (SEQ ID NO: 2).

2. The I-CreI homing endonuclease variant of claim 1 having a modified cleavage specificity, **characterized in that**:
when n₋₁₀n₋₉n₋₈ is aaa and n₋₅n₋₄n₋₃ is gtc in said DNA target then n₊₈n₊₉n₊₁0 is different from ttt and n+₃n₊₄n₊₅ is different from gac; and
when n+₈n₊₉n₊₁₀ is ttt and n₊₃n+₄n₊₅ is gac in said DNA target then n₋₁₀n₋₉n₋₈ is different from aaa and n₋₅n₋₄n₋₃ is different from gtc.

3. The I-CreI homing endonuclease variant of claim 1 or claim 2 having a modified cleavage specificity, **characterized in that** said modified cleavage specificity is towards at least one of the nucleotides in positions ±3 to ±5 and/or at least one of the nucleotides in positions ±8 to ±10 of said DNA target sequence consisting of a mutant I*-Cre*I*.*

4. The I-CreI homing endonuclease variant according to claim 3 having a modified cleavage specificity, **characterized in that** said DNA target sequence comprises a nucleotide triplet in positions -10 to -8 selected from the group consisting of : aac, aag, aat, acc, acg, act, aga, agc, agg, agt, ata, atg, cag, cga, cgg, ctg, gac, gag, gat, gaa, gcc, gga, ggc, ggg, ggt, gta, gtg, gtt, tac, tag, tat, tcc, tga, tgc, tgg, tgt, ttg, and / or a nucleotide triplet in positions +8 to +10, which is the reverse complementary sequence of said nucleotide triplet in positions -10 to -8.

5. The I-CreI homing endonuclease variant according to claim 3 having a modified cleavage specificity, **characterized in that** said variant comprises at least two mutations in the same *I-CreI* monomer, one of said mutations being in one of the amino acids residues in positions 26, 28, 30, 32, 33, 38 and 40 of I*-Cre*I and the other one of said mutations being in one of the amino acids residues in positions 44, 68, 70, 75 and/or 77 of I*-Cre*I.

6. The I*-Cre*I homing endonuclease variant according to claim 5 having at least the amino acid in positions 28, 30, 33, 38 and 40, respectively, selected in the group consisting of:
QNYKR, RNKRQ, QNRRR, QNYKK, QNTQK, QNRRK, KNTQR, SNRSR, NNYQR, KNTRQ, KNSRE, QNNQK, SNYRK, KNSRD, KNRER, KNSRS, RNRDR, ANSQR, QNYRK, QNKRT, RNAYQ, KNRQE, NNSRK, NNSRR, QNYQK, QNYQR, SNRQR, QNRQK, ENRRK, KNNQA, SNYQK, TNRQR, QNTQR, KNRTQ, KNRTR, QNEDH, RNYNA, QNYTR, RNTRA, HNYDS, QNYRA, QNYAR, SNQAA, QNYEK, TNNQR, QNYRS, KNRQR5 QNRAR, QNNQR, RNRER, KNRAR, KNTAA, KNRKA, RNAKS, KNRNA, TNESD, RNNQD, RNRYQ, KNYQN, KNRSS, KNRYA, ANNRK, KNRAT, KNRNQ, TNTQR, KNRQY, QNSRK, RNYQS, QNRQR, KNRAQ, ANRQR, KNRQQ, KNRQA, KNTAS, KAHRS, KHHRS, KDNHS, KESRS, KHTPS, KGHYS, KARQS, KSRGS, KSHHS, KNHRS, KRRES, KDGHS, KRHGS, KANQS, KDHKS, KKHRS, KQNQS, KQTQS, KGRQS, KRPGS, KRGNS, KNAQS, KNHNS, KHHAS, KRGSS, KSRQS, KTDHS, KHHQS, KADHS, KSHRS, KNRAS, KSHQS, KDAHS, KNHES, KDRTS, KDRSS, KAHQS3 KRGTS, KNHSS, KQHQS, KNHGS, KNNQS, KNDQS, KDRGS, KNHAS, KHMAS, KSSHS, KGVAS, KSVQS, KDVHS, RDVQS, KGVQS, KGVTS, KGVHS, KGVRS, KGVGS, RAVGS, RDVRS, RNVQS, and NTVDS.

7. The I*-Cre*I homing endonuclease variant according to claim 5 or claim 6, having at least the amino acids in positions 44, 68 and 70 selected in the group consisting of:
A44/A68/A70, A44/A68/G70, A44/A68/H70, A44/A68/K70, A44/A68/N70, A44/A68/Q70, A44/A68/R70, A44/A68/S70, A44/A68/T70, A44/D68/H70, A44/D68/K70, A44/D68/R70, A44/G68/H70, A44/G68/K70, A44/G68/N70, A44/G68/P70, A44/G68/R70, A44/H68/A70, A44/H68/G70, A44/H68/H70, A44/H68/K70, A44/H68/N70, A44/H68/Q70, A44/H68/R70, A44/H68/S70, A44/H68/T70, A44/K68/A70, A44/K68/G70, A44/K68/H70, A44/K68/K70, A44/K68/N70, A44/K68/Q70, A44/K68/R70, A44/K68/S70, A44/K68/T70, A44/N68/A70, A44/N68/E70, A44/N68/G70, A44/N68/H70, A44/N68/K70, A44/N68/N70, A44/N68/Q70, A44/N68/R70, A44/N68/570, A44/N68/T70, A44/Q68/A70, A44/Q68/D70, A44/Q68/G70, A44/Q68/H70, A44/Q68/N70, A44/Q68/R70, A44/Q68/S70, A44/R68/A70, A44/R68/D70, A44/R68/E70, A44/R68/G70, A44/R68/H70, A44/R68/K70, A44/R68/L70, A44/R68/N70, A44/R68/R70, A44/R68/S70, A44/R68/T70, A44/S68/A70, A44/S68/G70, A44/S68/K70, A44/S68/N70, A44/S68/Q70, A44/S68/R70, A44/S68/S70, A44/S68/T70, A44/T68/A70, A44/T68/G70, A44/T68/H70, A44/T68/K70, A44/T68/N70, A44/T68/Q70, A44/T68/R70, A44/T68/S70, A44/T68/T70, D44/D68/H70, D44/N68/S70, D44/R68/A70, D44/R68/K70, D44/R68/N70, D44/R68/Q70, D44/R68/R70, D44/R68/S70, D44/R68/T70, E44/H68/H70, E44/R68/A70, E44/R68/H70, E44/R68/N70, E44/R68/S70, E44/R68/T70, E44/S68/T70, G44/H68/K70, G44/Q68/H70, G44/R68/Q70, G44/R68/R70, G44/T68/D70, G44/T68/P70, G44/T68/R70, H44/A68/S70, H44/A68/T70, H44/R68/A70, H44/R68/D70, H44/R68/E70, H44/R68/G70, H44/R68/N70, H44/R68/R70, H44/R68/S70, H44/R68/T70, H44/S68/G70, H44/S68/S70, H44/S68/T70, H44/T68/S70, H44/T68/T70, K44/A68/A70, K44/A68/D70, K44/A68/E70, K44/A68/G70, K44/A68/H70, K44/A68/N70, K44/A68/Q70, K44/A68/S70, K44/A68/T70, K44/D68/A70, K44/D68/T70, K44/E68/G70, K44/E68/N70, K44/E68/S70, K44/G68/A70, K44/G68/G70, K44/G68/N70, K44/G68/S70, K44/G68/T70, K44/H68/D70, K44/H68/E70, K44/H68/G70, K44/H68/N70, K44/H68/S70, K44/H68/T70, K44/K68/A70, K44/K68/D70, K44/K68/H70, K44/K68/T70, K44/N68/A70, K44/N68/D70, K44/N68/E70, K44/N68/G70, K44/N68/H70, K44/N68/N70, K44/N68/Q70, K44/N68/S70, K44/N68/T70, K44/P68/H70, K44/Q68/A70, K44/Q68/D70, K44/Q68/E70, K44/Q68/S70, K44/Q68/T70, K44/R68/A70, K44/R68/D70, K44/R68/E70, K44/R68/G70, K44/R68/H70, K44/R68/N70, K44/R68/Q70, K44/R68/S70, K44/R68/T70, K44/S68/A70, K44/S68/D70, K44/S68/H70, K44/S68/N70, K44/S68/S70, K44/S68/T70, K44/T68/A70, K44/T68/D70, K44/T68/E70, K44/T68/G70, K44/T68/H70, K44/T68/N70, K44/T68/Q70, K44/T68/S70, K44/T68/T70, N44/A68/H70, N44/A68/R70, N44/H68/N70, N44/H68/R70, N44/K68/G70, N44/K68/H70, N44/K68/R70, N44/K68/S70, N44/N68/R70, N44/P68/D70, N44/Q68/H70, N44/Q68/R70, N44/R68/A70, N44/R68/D70, N44/R68/E70, N44/R68/G70, N44/R68/H70, N44/R68/K70, N44/R68/N70, N44/R68/R70, N44/R68/S70, N44/R68/T70, N44/S68/G70, N44/S68/H70, N44/S68/K70, N44/S68/R70, N44/T68/H70, N44/T68/K70, N44/T68/Q70, N44/T68/R70, N44/T68/S70, P44/N68/D70, P44/T68/T70, Q44/A68/A70, Q44/A68/H70, Q44/A68/R70, Q44/G68/K70, Q44/G68/R70, Q44/K68/G70, Q44/N68/A70, Q44/N68/H70, Q44/N68/S70, Q44/P68/P70, Q44/Q68/G70, Q44/R68/A70, Q44/R68/D70, Q44/R68/E70, Q44/R68/G70, Q44/R68/H70, Q44/R68/N70, Q44/R68/Q70, Q44/R68/S70, Q44/S68/H70, Q44/S68/R70, Q44/S68/S70, Q44/T68/A70, Q44/T68/G70, Q44/T68/H70, Q44/T68/R70, R44/A68/G70, R44/A68/T70, R44/G68/T70, R44/H68/D70, R44/H68/T70, R44/N68/T70, R44/R68/A70, R44/R68/D70, R44/R68/E70, R44/R68/G70, R44/R68/N70, R44/R68/Q70, R44/R68/S70, R44/R68/T70, R44/S68/G70, R44/S68/N70, R44/S68/S70, R44/S68/T70, S44/D68/K70, S44/H68/R70, S44/R68/G70, S44/R68/N70, S44/R68/R70, S44/R68/S70, T44/A68/K70, T44/A68/R70, T44/H68/R70, T44/K68/R70, T44/N68/P70, T44/N68/R70, T44/Q68/K70, T44/Q68/R70, T44/R68/A70, T44/R68/D70, T44/R68/E70, T44/R68/G70, T44/R68/H70, T44/R68/K70, T44/R68/N70, T44/R68/Q70, T44/R68/R70, T44/R68/S70, T44/R68/T70, T44/S68/K70, T44/S68/R70, T44/T68/K70, and T44/T68/R70.

8. The I-*Cre*I homing endonuclease variant according to anyone of claims 5 to 7, wherein said variant comprises at least one further mutation in at least one of positions 24, , 80, 82, 85, 86, 87, 94, 96, 100, 103, 114, 115, 117, 125, 129, 131, 132, 140, 147, 151, 153, 154, 155, 157, 159 and 160 of I*-Cre*I*.*

9. The I-*Cre*I homing endonuclease variant according to anyone of claims 1 to 8, **characterized in that** said variant is an homodimer.

10. The I-*Cre*I homing endonuclease variant according to anyone of claims 1 to 8, **characterized in that** said variant is an heterodimer.

11. The I-*Cre*I homing endonuclease variant according to claim 10, **characterized in that** the other monomer chosen for the formation of said heterodimer is from a LAGLIDADG homing endonuclease which is different from I*-Cre*I*.*

12. The I-CreI homing endonuclease variant according to anyone of claims 1 to 8, **characterized in that** said variant is a single-chain chimeric endonuclease.

13. The I*-Cre*I homing endonuclease variant according to claim 12, **characterized in that** said single-chain chimeric endonuclease comprises a I-CreI momomer variant as defined in any one of claims 1 to 8 and onother monomer from a LAGLIDADG homing endonuclease which is different from I*-Cre*I.

14. Use of a I-CreI homing endonuclease variant according to any one of claims 1 to 13, for the preparation of a medicament for preventing, improving or curing a disease caused by an infectious agent that presents a DNA intermediate, in an individual in need thereof, said medicament being for administration by any means to said individual.


SEQUENCE LISTING

<110> CELLECTIS
PAQUES, Frédéric

<120> LAGLIDADG HOMING ENDONUCLEASE VARIANTS HAVING MUTATIONS IN TWO
FUNCTIONAL SUBDOMAINS AND USE THEREOF

<130> 1546PCT7EXT

<160> 56

<170> PatentIn version 3.3

<210> 1
<211> 24
<212> DNA
<213> artificial sequence

<220>
<223> DNA target

<400> 1
tcaaaacgtc gtacgacgtt ttga 24

<210> 2
<211> 22
<212> DNA
<213> artificial sequence

<220>
<223> DNA target

<220>
<221> misc_feature
<222> (2)..(4)
<223> n is a, c, g, or t

<220>
<221> misc_feature
<222> (7)..(9)
<223> n is a, c, g, or t

<220>
<221> misc_feature
<222> (14)..(16)
<223> n is a, c, g, or t

<220>
<221> misc_feature
<222> (19)..(21)
<223> n is a, c, g, or t

<400> 2
cnnnmynnnk yrmnnnrknn ng 22

<210> 3
<211> 24
<212> DNA
<213> artificial sequence

<220>
<223> DNA target

<400> 3

tcaaaacgtt gtacaacgtt ttga 24

<210> 4
<211> 24
<212> DNA
<213> artificial sequence

<220>
<223> DNA target

<400> 4
tcaaaaccct gtacagggtt ttga 24

<210> 5
<211> 24
<212> DNA
<213> artificial sequence

<220>
<223> DNA target

<400> 5
tcaaaaccct gtacagggtt ttga 24

<210> 6
<211> 501
<212> DNA
<213> artificial sequence

<220>
<223> I-CreI N75 cDNA

<400> 6
atggccaata ccaaatataa caaagagttc ctgctgtacc tggccggctt tgtggacggt 60

gacggtagca tcatcgctca gattaaacca aaccagtctt ataagtttaa acatcagcta 120

agcttgacct ttcaggtgac tcaaaagacc cagcgccgtt ggtttctgga caaactagtg 180

gatgaaattg gcgttggtta cgtacgtgat cgcggatccg tttccaacta catcttaagc 240

gaaatcaagc cgctgcacaa cttcctgact caactgcagc cgtttctgaa actgaaacag 300

aaacaggcaa acctggttct gaaaattatc gaacagctgc cgtctgcaaa agaatccccg 360

gacaaattcc tggaagtttg tacctgggtg gatcagattg cagctctgaa cgattctaag 420

acgcgtaaaa ccacttctga aaccgttcgt gctgtgctgg acagcctgag cgagaagaag 480

aaatcctccc cggcggccga c 501

<210> 7
<211> 68
<212> DNA
<213> artificial sequence

<220>
<223> primer

<400> 7
ctgaaaggtc aagcttagmb batgtttaaa cttmbbagac tgmbbtggtt taatctgagc 60

gatgatgc 68

<210>  8
<211>  68
<212>  DNA
<213>  artificial sequence

<220>
<223>  primer

<400>  8
ctgaaaggtc aagcttagmb batgtttaaa cttataagac tgmbbtggmb baatctgagc      60

gatgatgc                                                                68


<210>  9
<211>  18
<212>  DNA
<213>  artificial sequence

<220>
<223>  primer

<400>  9
ctaagcttga cctttcag                                                     18


<210>  10
<211>  24
<212>  DNA
<213>  artificial sequence

<220>
<223>  DNA target

<400>  10
tcaaaacggg gtaccccgtt ttga                                              24


<210>  11
<211>  24
<212>  DNA
<213>  artificial sequence

<220>
<223>  DNA target

<400>  11
tcaaaacgga gtactccgtt ttga                                              24


<210>  12
<211>  24
<212>  DNA
<213>  artificial sequence

<220>
<223>  DNA target

<400>  12
tcaaaacggt gtacaccgtt ttga                                              24


<210>  13
<211>  24
<212>  DNA
<213>  artificial sequence

<220>
<223>  DNA target

<400>  13
tcaaaacggc gtacgccgtt ttga                                              24


<210>  14
<211>  24
<212>  DNA
<213>  artificial sequence

<220>
<223>  DNA target

<400>  14
tcaaaacgag gtacctcgtt ttga                                              24


<210>  15
<211>  24
<212>  DNA
<213>  artificial sequence

<220>
<223>  DNA target

<400>  15
tcaaaacgaa gtacttcgtt ttga                                              24


<210>  16
<211>  24
<212>  DNA
<213>  artificial sequence

<220>
<223>  DNA target

<400>  16
tcaaaacgat gtacatcgtt ttga                                              24


<210>  17
<211>  24
<212>  DNA
<213>  artificial sequence

<220>
<223>  DNA target

<400>  17
tcgggacgtc gtacgacgtc ccga                                              24


<210>  18
<211>  24
<212>  DNA
<213>  artificial sequence

<220>
<223>  DNA target

<400>  18
tcggaacgtc gtacgacgtt ccga                                              24


<210>  19

<211>  24
<212>  DNA
<213>  artificial sequence

<220>
<223>  DNA target

<400>  19
tcggtacgtc gtacgacgta ccga                                              24


<210>  20
<211>  24
<212>  DNA
<213>  artificial sequence

<220>
<223>  DNA target

<400>  20
tcggcacgtc gtacgacgtg ccga                                              24


<210>  21
<211>  24
<212>  DNA
<213>  artificial sequence

<220>
<223>  DNA target

<400>  21
tcgagacgtc gtacgacgtc tcga                                              24


<210>  22
<211>  24
<212>  DNA
<213>  artificial sequence

<220>
<223>  DNA target

<400>  22
tcgaaacgtc gtacgacgtt tcga                                              24


<210>  23
<211>  24
<212>  DNA
<213>  artificial sequence

<220>
<223>  DNA target

<400>  23
tcgatacgtc gtacgacgta tcga                                              24


<210>  24
<211>  24
<212>  DNA
<213>  artificial sequence

<220>
<223>  DNA target

<400>  24

tcaaaacgtc gtgagacagt ttgg                                          24

<210>  25
<211>  49
<212>  DNA
<213>  artificial sequence

<220>
<223>  oligonucleotide

<220>
<221>  misc_feature
<222>  (20)..(22)
<223>  n is a, c, g, or t

<220>
<221>  misc_feature
<222>  (27)..(29)
<223>  n is a, c, g, or t

<400>  25
ggcatacaag tttcaaaacn nngtacnnng ttttgacaat cgtctgtca                 49

<210>  26
<211>  77
<212>  DNA
<213>  artificial sequence

<220>
<223>  primer

<400>  26
ggggacaagt ttgtacaaaa aagcaggctt cgaaggagat agaaccatgg ccaataccaa     60

atataacaaa gagttcc                                                    77

<210>  27
<211>  64
<212>  DNA
<213>  artificial sequence

<220>
<223>  primer

<400>  27
ggggaccact ttgtacaaga aagctgggtt tagtcggccg ccggggagga tttcttcttc     60

tcgc                                                                  64

<210>  28
<211>  49
<212>  DNA
<213>  artificial sequence

<220>
<223>  DNA target

<220>
<221>  misc_feature
<222>  (15)..(17)
<223>  n is a, c, g, or t

<220>
<221> misc_feature
<222> (32)..(34)
<223> n is a, c, g, or t

<400> 28
ggcatacaag tttcnnnacg tcgtacgacg tnnngacaat cgtctgtca 49

<210> 29
<211> 26
<212> DNA
<213> artificial sequence

<220>
<223> primer

<400> 29
gcaactttag tgctgacaca tacagg 26

<210> 30
<211> 24
<212> DNA
<213> artificial sequence

<220>
<223> primer

<400> 30
acaaccttga ttgcagactt gacc 24

<210> 31
<211> 49
<212> DNA
<213> artificial sequence

<220>
<223> oligoucleotide

<220>
<221> misc_feature
<222> (20)..(22)
<223> n is a, c, g, or t

<220>
<221> misc_feature
<222> (27)..(29)
<223> n is a, c, g, or t

<400> 31
ggcatacaag tttcaaaacn nngtacnnng ttttgacaat cgtctgtca 49

<210> 32
<400> 32
000

<210> 33
<400> 33
000

<210> 34
<400> 34
000

<210> 35

<400>  35
000

<210>  36
<400>  36
000

<210>  37
<211>  12
<212>  DNA
<213>  artificial sequence

<220>
<223>  DNA target

<400>  37
tctggacgtc gt                                                          12


<210>  38
<211>  12
<212>  DNA
<213>  artificial sequence

<220>
<223>  DNA target

<400>  38
tcaaaacgac gt                                                          12


<210>  39
<211>  12
<212>  DNA
<213>  artificial sequence

<220>
<223>  DNA target

<400>  39
tctggacgac gt                                                          12


<210>  40
<211>  26
<212>  DNA
<213>  artificial sequence

<220>
<223>  primer

<400>  40
gcaactttag tgctgacaca tacagg                                           26


<210>  41
<211>  24
<212>  DNA
<213>  artificial sequence

<220>
<223>  primer

<400>  41
acaaccttga ttggagactt gacc                                             24


<210>  42

<211>  15
<212>  DNA
<213>  artificial sequence

<220>
<223>  primer


<220>
<221>  misc_feature
<222>  (4)..(6)
<223>  n is a, c, g, or t

<400>  42
ctannnttga ccttt                                                        15


<210>  43
<211>  15
<212>  DNA
<213>  artificial sequence

<220>
<223>  primer


<220>
<221>  misc_feature
<222>  (10)..(12)
<223>  n is a, c, g, or t

<400>  43
aaaggtcaan nntag                                                        15


<210>  44
<211>  12
<212>  DNA
<213>  artificial sequence

<220>
<223>  DNA target

<400>  44
tcgatacgtc gt                                                           12


<210>  45
<211>  12
<212>  DNA
<213>  artificial sequence

<220>
<223>  DNA target

<400>  45
tcaaaaccct gt                                                           12


<210>  46
<211>  12
<212>  DNA
<213>  artificial sequence

<220>
<223>  DNA target

<400>  46

tcgataccct gt                                                          12

<210>  47
<211>  12
<212>  DNA
<213>  artificial sequence

<220>
<223>  DNA target

<400>  47
tcaacacgtc gt                                                          12


<210>  48
<211>  12
<212>  DNA
<213>  artificial sequence

<220>
<223>  DNA target

<400>  48
tcaaaaccct gt                                                          12


<210>  49
<211>  12
<212>  DNA
<213>  artificial sequence

<220>
<223>  DNA target

<400>  49
tcaacaccct gt                                                          12


<210>  50
<211>  12
<212>  DNA
<213>  artificial sequence

<220>
<223>  DNA target

<400>  50
tcaacacgtc gt                                                          12


<210>  51
<211>  12
<212>  DNA
<213>  artificial sequence

<220>
<223>  DNA target

<400>  51
tcaaaacttt gt                                                          12


<210>  52
<211>  12
<212>  DNA
<213>  artificial sequence

<220>
<223>  DNA target

<400>  52
tcaacacttt gt                                                          12


<210>  53
<211>  22
<212>  DNA
<213>  artificial sequence

<220>
<223>  DNA target

<400>  53
ctggacgacg tacagggtat cg                                               22


<210>  54
<211>  22
<212>  DNA
<213>  artificial sequence

<220>
<223>  DNA target

<400>  54
tgttctcagg tacctcagcc ag                                               22


<210>  55
<211>  22
<212>  DNA
<213>  artificial sequence

<220>
<223>  DNA target

<400>  55
tgttctcagg tacctgagaa ca                                               22


<210>  56
<211>  22
<212>  DNA
<213>  artificial sequence

<220>
<223>  DNA target

<400>  56
ctggctgagg tacctcagcc ag                                               22